# EUROPEAN PATENT APPLICATION

(11) **EP 4 667 491 A1**
(43) Date of publication of application: **24.12.2025**
(21) Application number: 24757093.0
(22) Date of filing: 02.02.2024
(51) Int. Cl.: C07K 16/28, A61K 47/68, A61P 35/00, A61K 39/00

(54) **ANTI-CNTN4 ANTIBODY AND USES THEREOF**

(30) Priority: 15.02.2023 KR 20230020056
(71) Applicant: Genome and Company, Seongnam-si, Gyeonggi-do 13486 (KR)
(72) Inventor: CHA, Mi Young, Seongnam-si, Gyeonggi-do 13486 (KR); KIM, Hyunuk, Seongnam-si, Gyeonggi-do 13486 (KR); YU, Hyunkyung, Seongnam-si, Gyeonggi-do 13486 (KR); KIM, Yun Yeon, Seongnam-si, Gyeonggi-do 13486 (KR); JEON, Bunam, Seongnam-si, Gyeonggi-do 13486 (KR); HA, Youngeun, Seongnam-si, Gyeonggi-do 13486 (KR)
(74) Representative: Abel & Imray LLP
(86) International application number: PCT/KR2024/001620
(87) International publication number: WO 2024/172363

(57) **Abstract**

The present invention relates to an anti-CNTN4 antibody or an antigen-binding fragment thereof, and uses thereof.

## Description

### Technical Field

The present invention relates to an anti-CNTN4 antibody or an antigen-binding fragment thereof, uses thereof, and a pharmaceutical composition including the same.

### Background Art

The human body has a defense system that protects itself from external invaders (viruses, toxins, etc.) and internal hazardous changes (cancer cell mutations). Cancer cells, unlike normal cells, have specific antigens on their surface, so they are destroyed by the immune system in the early stage of cancer development. Then, when the balance of power between indefinitely proliferating cancer cells and immune cells that attack them is disturbed, the cancer cells begin to proliferate in earnest. When cancer cells continue to grow, they disrupt the body's immune system. At this time, some cancer cells evade immunity by utilizing the immune checkpoints of immune cells, and immune checkpoint inhibitors block these checkpoints, enhancing the power of immune cells, resulting in cancer cell death.

Immune checkpoints are a group of signal molecules possessed by immune cells, and immune checkpoint proteins are proteins involved in a pathway that inhibits immune responses and proteins involved in a pathway that activates immune responses.

Immune checkpoint inhibitors are drugs that activate T cells to attack cancer cells by blocking the activation of immune checkpoint proteins involved in T cell suppression, and include CTLA-4, PD-1, and PD-L1 inhibitors. Currently available representative drugs include ipilimumab (product name: YERVOY^{®}) as a CTLA-4 monoclonal antibody, nivolumab (product name: OPDIVO^{®}) and pembrolizumab (product name: KEYTRUDA^{®}) as PD-1 monoclonal antibodies, and atezolizumab (product name: TECENTRIQ^{®}) and durvalumab (product name: IMFINZI^{®}) as PD-L1 monoclonal antibodies.

However, there are still cancers that cannot be treated by conventional immune checkpoint inhibitors, and thus there is a need for the development of a novel anticancer therapeutic agent.

### Detailed Description of Invention

### Technical Problem

The present invention is directed to providing an anti-CNTN4 antibody or an antigen-binding fragment thereof, which specifically binds to a CNTN4 protein. Particularly, the present invention is directed to providing an antibody or an antigen-binding fragment thereof, which binds to a CNTN4 protein to neutralize the immune evasion mechanism of CNTN4.

The present invention is also directed to providing a use for preventing or treating a disease caused by reduced T cell activity, for example, cancer or a composition for preventing or treating cancer, by activating T cells by blocking the immune evasion mechanism of CNTN4 using the antibody or an antigen-binding fragment thereof.

### Technical Solution

The present invention provides an anti-CNTN4 antibody or an antigen-binding fragment thereof, which specifically binds to a CNTN4 protein. The antibody or an antigen-binding fragment specifically binds to a CNTN4 protein, for example, a human or mouse CNTN4 protein to neutralize the immune evasion mechanism of CNTN4. Accordingly, the antibody or an antigen-binding fragment of the present invention may enhance the activity of T cells suppressed by CNTN4, for example, CD4+T cells or CD8+T cells.

In one embodiment, the present invention provides an anti-CNTN4 antibody or an antigen-binding fragment thereof, which includes:
heavy chain CDR1 having the amino acid sequence of SEQ ID NO: 1;
heavy chain CDR2 having the amino acid sequence of SEQ ID NO: 4;
heavy chain CDR3 having any one amino acid sequence selected from the group consisting of SEQ ID NOs: 6, 8 to 18, and 21 to 35;
light chain CDR1 having the amino acid sequence of SEQ ID NO: 2 or 3;
light chain CDR2 having the amino acid sequence of SEQ ID NO: 5; and
light chain CDR3 having any one amino acid sequence selected from the group consisting of SEQ ID NOs: 7, 19, and 20.

In one embodiment, the present invention provides an anti-CNTN4 antibody or an antigen-binding fragment thereof, including:
a heavy chain variable region having any one amino acid sequence selected from the group consisting of SEQ ID NOs: 36, 38 to 48, 52, and 54 to 67; and
a light chain variable region having any one amino acid sequence selected from the group consisting of SEQ ID NOs: 37, 49 to 51, and 53.

In one embodiment, the antigen-binding fragment of the present invention may be Fab, Fab', Fab'-SH, Fv, a single-chain variable fragment (scFv), an F(ab')2 fragment, VL, VH, a diabody, a triabody, a tetrabody, a minibody ((scFV-CH3)2), IgG ΔCH2, scFv-Fc, (scFv)2-Fc, a fynomer, dual-affinity re-targeting (DART), or TRIDENT, which includes a CDR sequence of the present invention. The antibody of the present invention may be a chimeric antibody, a humanized antibody, or a human antibody, and a multispecific antibody.

In another embodiment, the present invention provides a nucleic acid molecule encoding the antibody or an antigen-binding fragment thereof, and a recombinant expression vector including the same.

The present invention also provides a composition for preventing or treating cancer, which includes the antibody or an antigen-binding fragment thereof as an active ingredient. The pharmaceutical composition may be used in combination with an additional anticancer agent, for example, an immune checkpoint inhibitor or a chemotherapeutic agent, or with radiotherapy.

The present invention also provides a composition for analyzing or detecting a CNTN4 protein, which includes the antibody or an antigen-binding fragment thereof.

### Advantageous Effects

The novel anti-CNTN4 antibody or an antigen-binding fragment thereof of the present invention binds to the CNTN4 protein with high affinity. Therefore, the anti-CNTN4 antibody or an antigen-binding fragment thereof of the present invention can be useful in prevention or treatment of a disease caused by reduced T cell activity, particularly, cancer, by activating T cells by blocking the immune evasion mechanism of CNTN4.

### Description of Drawings

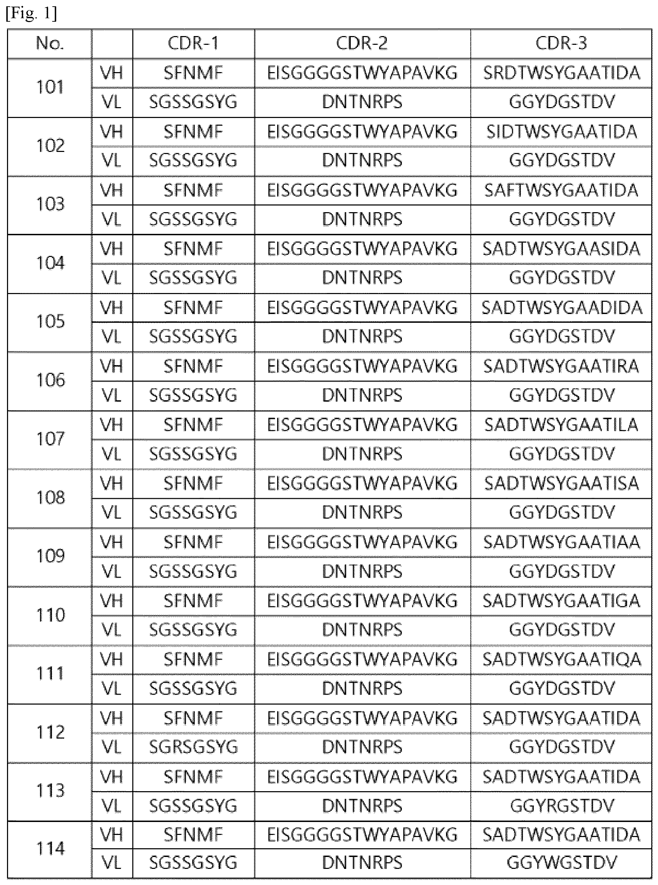

FIGS. 1 and 2 show the amino acid sequences of CDRs of the scFv antibody fragment prepared according to Example 1 (FIG. 1), and the amino acid sequences of heavy chain and light chain variable regions thereof (FIG. 2: the sequence underlined or in bold indicates the CDR sequence of each region).
FIG. 3 shows data (MFI) obtained by confirming the binding degrees of nine IgG antibodies (A102, A103, A104, A105, A106, A108, A109, A112, and A114) to HEK293 cells overexpressing human CNTN4 by concentration through an FACS experiment.
FIGS. 4 and 5 show the amino acid sequences of CDRs of the scFv antibody fragment prepared according to Example 4 (FIG. 4), and the amino acid sequences of heavy chain and light chain variable regions thereof (FIG. 5: the sequence underlined or in bold indicates the CDR sequence of each region).
FIG. 6 shows data (MFI) obtained by confirming the binding degrees of A114-IgG and A211-IgG antibodies to HEK293 cells overexpressing human CNTN4 by concentration through an FACS experiment.
FIG. 7 shows the absolute tumor size (FIG. 7A) and relative tumor size compared to a control (FIG. 7B) after administering an A114-IgG antibody and an A211-IgG antibody.

### Best Mode

### Anti-CNTN4 antibody or antigen-binding fragment thereof

The present invention provides an anti-CNTN4 antibody or an antigen-binding fragment thereof, which specifically binds to a CNTN4 protein.

In one embodiment, the anti-CNTN4 antibody or an antigen-binding fragment thereof of the present invention includes
heavy chain CDR1 having the amino acid sequence of SEQ ID NO: 1;
heavy chain CDR2 having the amino acid sequence of SEQ ID NO: 4;
heavy chain CDR3 having any one amino acid sequence selected from the group consisting of SEQ ID NOs: 6, 8 to 18, and 21 to 35;
light chain CDR1 having the amino acid sequence of SEQ ID NO: 2 or 3;
light chain CDR2 having the amino acid sequence of SEQ ID NO: 5; and
light chain CDR3 having any one amino acid sequence selected from the group consisting of SEQ ID NOs: 7, 19, and 20.

In still another embodiment, the anti-CNTN4 antibody or an antigen-binding fragment thereof of the present invention may include one or more CDRs of the CDR1 to CDR3 of the heavy chains or light chains described above.

In an exemplary embodiment, the anti-CNTN4 antibody or an antigen-binding fragment thereof of the present invention may include one or more CDRs among the set of 6 CDRs of the heavy and light chains shown in Tables 1 and 4, or all 6 CDRs thereof.

In an embodiment, the anti-CNTN4 antibody or an antigen-binding fragment thereof of the present invention includes
101) heavy chain CDR1, heavy chain CDR2, heavy chain CDR3, light chain CDR1, light chain CDR2, and light chain CDR3, which include the amino acid sequences of SEQ ID NOs: 1, 4, 6, 2, 5, and 7, respectively;
102) heavy chain CDR1, heavy chain CDR2, heavy chain CDR3, light chain CDR1, light chain CDR2, and light chain CDR3, which include the amino acid sequences of SEQ ID NOs: 1, 4, 8, 2, 5, and 7, respectively;
103) heavy chain CDR1, heavy chain CDR2, heavy chain CDR3, light chain CDR1, light chain CDR2, and light chain CDR3, which include the amino acid sequences of SEQ ID NOs: 1, 4, 9, 2, 5, and 7, respectively;
104) heavy chain CDR1, heavy chain CDR2, heavy chain CDR3, light chain CDR1, light chain CDR2, and light chain CDR3, which include the amino acid sequences of SEQ ID NOs: 1, 4, 10, 2, 5, and 7, respectively;
105) heavy chain CDR1, heavy chain CDR2, heavy chain CDR3, light chain CDR1, light chain CDR2, and light chain CDR3, which include the amino acid sequences of SEQ ID NOs: 1, 4, 11, 2, 5, and 7, respectively;
106) heavy chain CDR1, heavy chain CDR2, heavy chain CDR3, light chain CDR1, light chain CDR2, and light chain CDR3, which include the amino acid sequences of SEQ ID NOs: 1, 4, 12, 2, 5, and 7, respectively;
107) heavy chain CDR1, heavy chain CDR2, heavy chain CDR3, light chain CDR1, light chain CDR2, and light chain CDR3, which include the amino acid sequences of SEQ ID NOs: 1, 4, 13, 2, 5, and 7, respectively;
108) heavy chain CDR1, heavy chain CDR2, heavy chain CDR3, light chain CDR1, light chain CDR2, and light chain CDR3, which include the amino acid sequences of SEQ ID NOs: 1, 4, 14, 2, 5, and 7, respectively;
109) heavy chain CDR1, heavy chain CDR2, heavy chain CDR3, light chain CDR1, light chain CDR2, and light chain CDR3, which include the amino acid sequences of SEQ ID NOs: 1, 4, 15, 2, 5, and 7, respectively;
110) heavy chain CDR1, heavy chain CDR2, heavy chain CDR3, light chain CDR1, light chain CDR2, and light chain CDR3, which include the amino acid sequences of SEQ ID NOs: 1, 4, 16, 2, 5, and 7, respectively;
111) heavy chain CDR1, heavy chain CDR2, heavy chain CDR3, light chain CDR1, light chain CDR2, and light chain CDR3, which include the amino acid sequences of SEQ ID NOs: 1, 4, 17, 2, 5, and 7, respectively;
112) heavy chain CDR1, heavy chain CDR2, heavy chain CDR3, light chain CDR1, light chain CDR2, and light chain CDR3, which include the amino acid sequences of SEQ ID NOs: 1, 4, 18, 3, 5, and 7, respectively;
113) heavy chain CDR1, heavy chain CDR2, heavy chain CDR3, light chain CDR1, light chain CDR2, and light chain CDR3, which include the amino acid sequences of SEQ ID NOs: 1, 4, 18, 2, 5, and 19, respectively;
114) heavy chain CDR1, heavy chain CDR2, heavy chain CDR3, light chain CDR1, light chain CDR2, and light chain CDR3, which include the amino acid sequences of SEQ ID NOs: 1, 4, 18, 2, 5, and 20, respectively;
201) heavy chain CDR1, heavy chain CDR2, heavy chain CDR3, light chain CDR1, light chain CDR2, and light chain CDR3, which include the amino acid sequences of SEQ ID NOs: 1, 4, 21, 3, 5, and 20, respectively;
202) heavy chain CDR1, heavy chain CDR2, heavy chain CDR3, light chain CDR1, light chain CDR2, and light chain CDR3, which include the amino acid sequences of SEQ ID NOs: 1, 4, 22, 3, 5, and 20, respectively;
203) heavy chain CDR1, heavy chain CDR2, heavy chain CDR3, light chain CDR1, light chain CDR2, and light chain CDR3, which include the amino acid sequences of SEQ ID NOs: 1, 4, 23, 3, 5, and 20, respectively;
204) heavy chain CDR1, heavy chain CDR2, heavy chain CDR3, light chain CDR1, light chain CDR2, and light chain CDR3, which include the amino acid sequences of SEQ ID NOs: 1, 4, 24, 3, 5, and 20, respectively;
205) heavy chain CDR1, heavy chain CDR2, heavy chain CDR3, light chain CDR1, light chain CDR2, and light chain CDR3, which include the amino acid sequences of SEQ ID NOs: 1, 4, 21, 2, 5, and 20, respectively;
206) heavy chain CDR1, heavy chain CDR2, heavy chain CDR3, light chain CDR1, light chain CDR2, and light chain CDR3, which include the amino acid sequences of SEQ ID NOs: 1, 4, 25, 3, 5, and 20, respectively;
207) heavy chain CDR1, heavy chain CDR2, heavy chain CDR3, light chain CDR1, light chain CDR2, and light chain CDR3, which include the amino acid sequences of SEQ ID NOs: 1, 4, 26, 2, 5, and 20, respectively;
208) heavy chain CDR1, heavy chain CDR2, heavy chain CDR3, light chain CDR1, light chain CDR2, and light chain CDR3, which include the amino acid sequences of SEQ ID NOs: 1, 4, 27, 2, 5, and 20, respectively;
209) heavy chain CDR1, heavy chain CDR2, heavy chain CDR3, light chain CDR1, light chain CDR2, and light chain CDR3, which include the amino acid sequences of SEQ ID NOs: 1, 4, 26, 3, 5, and 20, respectively;
210) heavy chain CDR1, heavy chain CDR2, heavy chain CDR3, light chain CDR1, light chain CDR2, and light chain CDR3, which include the amino acid sequences of SEQ ID NOs: 1, 4, 28, 3, 5, and 20, respectively;
211) heavy chain CDR1, heavy chain CDR2, heavy chain CDR3, light chain CDR1, light chain CDR2, and light chain CDR3, which include the amino acid sequences of SEQ ID NOs: 1, 4, 27, 3, 5, and 20, respectively;
212) heavy chain CDR1, heavy chain CDR2, heavy chain CDR3, light chain CDR1, light chain CDR2, and light chain CDR3, which include the amino acid sequences of SEQ ID NOs: 1, 4, 9, 3, 5, and 20, respectively;
213) heavy chain CDR1, heavy chain CDR2, heavy chain CDR3, light chain CDR1, light chain CDR2, and light chain CDR3, which include the amino acid sequences of SEQ ID NOs: 1, 4, 29, 2, 5, and 20, respectively;
214) heavy chain CDR1, heavy chain CDR2, heavy chain CDR3, light chain CDR1, light chain CDR2, and light chain CDR3, which include the amino acid sequences of SEQ ID NOs: 1, 4, 30, 2, 5, and 20, respectively;
215) heavy chain CDR1, heavy chain CDR2, heavy chain CDR3, light chain CDR1, light chain CDR2, and light chain CDR3, which include the amino acid sequences of SEQ ID NOs: 1, 4, 31, 2, 5, and 20, respectively;
216) heavy chain CDR1, heavy chain CDR2, heavy chain CDR3, light chain CDR1, light chain CDR2, and light chain CDR3, which include the amino acid sequences of SEQ ID NOs: 1, 4, 32, 3, 5, and 20, respectively;
217) heavy chain CDR1, heavy chain CDR2, heavy chain CDR3, light chain CDR1, light chain CDR2, and light chain CDR3, which include the amino acid sequences of SEQ ID NOs: 1, 4, 33, 2, 5, and 20, respectively;
218) heavy chain CDR1, heavy chain CDR2, heavy chain CDR3, light chain CDR1, light chain CDR2, and light chain CDR3, which include the amino acid sequences of SEQ ID NOs: 1, 4, 34, 3, 5, and 20, respectively; or
219) heavy chain CDR1, heavy chain CDR2, heavy chain CDR3, light chain CDR1, light chain CDR2, and light chain CDR3, which include the amino acid sequences of SEQ ID NOs: 1, 4, 35, 2, 5, and 20, respectively.

In the present invention, each chain or a variable region "including" a specific amino acid sequence means including the entire amino acid sequence, having the entire amino acid sequence, or consisting entirely of the amino acid sequence.

In one example, the antibody or an antigen-binding fragment thereof includes heavy chain CDR1 having the amino acid sequence of SEQ ID NO: 1; heavy chain CDR2 having the amino acid sequence of SEQ ID NO: 4; heavy chain CDR3 having the amino acid sequence of SEQ ID NO: 6; light chain CDR1 having the amino acid sequence of SEQ ID NO: 2; light chain CDR2 having the amino acid sequence of SEQ ID NO: 5; and light chain CDR3 having the amino acid sequence of SEQ ID NO: 7.

In another example, the antibody or an antigen-binding fragment thereof includes heavy chain CDR1 essentially consisting of the amino acid sequence of SEQ ID NO: 1; heavy chain CDR2 essentially consisting of the amino acid sequence of SEQ ID NO: 4; heavy chain CDR3 essentially consisting of the amino acid sequence of SEQ ID NO: 6; light chain CDR1 essentially consisting of the amino acid sequence of SEQ ID NO: 2; light chain CDR2 essentially consisting of the amino acid sequence of SEQ ID NO: 5; and light chain CDR3 essentially consisting of the amino acid sequence of SEQ ID NO: 7.

In still another example, the antibody or an antigen-binding fragment thereof includes heavy chain CDR1 consisting of the amino acid sequence of SEQ ID NO: 1; heavy chain CDR2 consisting of the amino acid sequence of SEQ ID NO: 4; heavy chain CDR3 consisting of the amino acid sequence of SEQ ID NO: 6; light chain CDR1 consisting of the amino acid sequence of SEQ ID NO: 2; light chain CDR2 consisting of the amino acid sequence of SEQ ID NO: 5; and light chain CDR3 consisting of the amino acid sequence of SEQ ID NO: 7.

The term "including," "include," or variations thereof have open-ended meanings. In one example, the antibodies or antigen-binding fragments thereof, including the above-listed amino acid sequences, may include an additional amino acid sequence, which is not listed above, regardless of whether it is essential.

The term "essentially consisting of" used herein or variations thereof includes any of the cited elements and allows the presence of elements that do not substantially affect the fundamental, novel, or functional characteristics of the embodiment. In one example, the antibodies or antigen-binding fragments thereof essentially consisting of the listed amino acid sequences may include substitutions of one or more amino acid residues that do not substantially affect the characteristics of the antibodies or fragments thereof.

The term "consisting of" used herein or variations thereof means a case where each component does not allow any element that is not mentioned or listed in the description of the embodiment.

Each chain or variable region of another antibody or an antigen-binding fragment thereof defined herein may similarly include, or may be essentially constituted by or consist of an amino acid sequence as exemplified above.

The term "complementarity-determining region" or "CDR" refers to amino acid residues in a variable region of an antibody, which are essential for antigen binding. Each variable region has typically three CDR regions identified as CDR1, CDR2, and CDR3. CDRs include most residues that mediate a specific interaction between an antibody (or its antigen-binding fragment) and an antigen, and thus contributes to the functional activation of an antibody molecule. These are the main determinants of antigen specificity.

In yet another embodiment, the anti-CNTN4 antibody or an antigen-binding fragment thereof of the present invention includes a heavy chain variable region having any one amino acid sequence selected from the group consisting of SEQ ID NOs: 36, 38 to 48, 52, and 54 to 67; and
a light chain variable region having any one amino acid sequence selected from the group consisting of SEQ ID NOs: 37, 49 to 51, and 53.

In yet another embodiment, the anti-CNTN4 antibody or an antigen-binding fragment thereof of the present invention may include one or more variable regions of the above-mentioned heavy chain variable regions and light chain variable regions.

In an exemplary embodiment, the anti-CNTN4 antibody or an antigen-binding fragment thereof of the present invention may include one or more variable regions of pairs of one heavy chain variable region and one light chain variable region mentioned in Tables 2 and 5. In a specific example, the antibody or an antigen-binding fragment thereof of the present invention may include
A101) a heavy chain variable region and a light chain variable region, which have the amino acid sequences of SEQ ID NOs: 36 and 37, respectively;
A102) a heavy chain variable region and a light chain variable region, which have the amino acid sequences of SEQ ID NOs: 38 and 37, respectively;
A103) a heavy chain variable region and a light chain variable region, which have the amino acid sequences of SEQ ID NOs: 39 and 37, respectively;
A104) a heavy chain variable region and a light chain variable region, which have the amino acid sequences of SEQ ID NOs: 40 and 37, respectively;
A105) a heavy chain variable region and a light chain variable region, which have the amino acid sequences of SEQ ID NOs: 41 and 37, respectively;
A106) a heavy chain variable region and a light chain variable region, which have the amino acid sequences of SEQ ID NOs: 42 and 37, respectively;
A107) a heavy chain variable region and a light chain variable region, which have the amino acid sequences of SEQ ID NOs: 43 and 37, respectively;
A108) a heavy chain variable region and a light chain variable region, which have the amino acid sequences of SEQ ID NOs: 44 and 37, respectively;
A109) a heavy chain variable region and a light chain variable region, which have the amino acid sequences of SEQ ID NOs: 45 and 37, respectively;
A110) a heavy chain variable region and a light chain variable region, which have the amino acid sequences of SEQ ID NOs: 46 and 37, respectively;
A111) a heavy chain variable region and a light chain variable region, which have the amino acid sequences of SEQ ID NOs: 47 and 37, respectively;
A112) a heavy chain variable region and a light chain variable region, which have the amino acid sequences of SEQ ID NOs: 48 and 49, respectively;
A113) a heavy chain variable region and a light chain variable region, which have the amino acid sequences of SEQ ID NOs: 48 and 50, respectively;
A114) a heavy chain variable region and a light chain variable region, which have the amino acid sequences of SEQ ID NOs: 48 and 51, respectively;
A201) a heavy chain variable region and a light chain variable region, which have the amino acid sequences of SEQ ID NOs: 52 and 53, respectively;
A202) a heavy chain variable region and a light chain variable region, which have the amino acid sequences of SEQ ID NOs: 54 and 53, respectively;
A203) a heavy chain variable region and a light chain variable region, which have the amino acid sequences of SEQ ID NOs: 55 and 53, respectively;
A204) a heavy chain variable region and a light chain variable region, which have the amino acid sequences of SEQ ID NOs: 56 and 53, respectively;
A205) a heavy chain variable region and a light chain variable region, which have the amino acid sequences of SEQ ID NOs: 52 and 51, respectively;
A206) a heavy chain variable region and a light chain variable region, which have the amino acid sequences of SEQ ID NOs: 57 and 53, respectively;
A207) a heavy chain variable region and a light chain variable region, which have the amino acid sequences of SEQ ID NOs: 58 and 51, respectively;
A208) a heavy chain variable region and a light chain variable region, which have the amino acid sequences of SEQ ID NOs: 59 and 51, respectively;
A209) a heavy chain variable region and a light chain variable region, which have the amino acid sequences of SEQ ID NOs: 58 and 53, respectively;
A210) a heavy chain variable region and a light chain variable region, which have the amino acid sequences of SEQ ID NOs: 60 and 53, respectively;
A211) a heavy chain variable region and a light chain variable region, which have the amino acid sequences of SEQ ID NOs: 59 and 53, respectively;
A212) a heavy chain variable region and a light chain variable region, which have the amino acid sequences of SEQ ID NOs: 39 and 53, respectively;
A213) a heavy chain variable region and a light chain variable region, which have the amino acid sequences of SEQ ID NOs: 61 and 51, respectively;
A214) a heavy chain variable region and a light chain variable region, which have the amino acid sequences of SEQ ID NOs: 62 and 51, respectively;
A215) a heavy chain variable region and a light chain variable region, which have the amino acid sequences of SEQ ID NOs: 63 and 51, respectively;
A216) a heavy chain variable region and a light chain variable region, which have the amino acid sequences of SEQ ID NOs: 64 and 53, respectively;
A217) a heavy chain variable region and a light chain variable region, which have the amino acid sequences of SEQ ID NOs: 65 and 51, respectively;
A218) a heavy chain variable region and a light chain variable region, which have the amino acid sequences of SEQ ID NOs: 66 and 53, respectively; or
A219) a heavy chain variable region and a light chain variable region, which have the amino acid sequences of SEQ ID NOs: 67 and 51,
   respectively.

In the same manner as defined above, in the present invention, the heavy chain or light chain variable region "including" a specific amino acid sequence means including the entire amino acid sequence, having the entire amino acid sequence, or consisting entirely of the amino acid sequence.

Accordingly, in one example, the antibody or an antigen-binding fragment thereof of the present invention includes a heavy chain variable region having the amino acid sequence of SEQ ID NO: 36 and a light chain variable region having the amino acid sequence of SEQ ID NO: 37.

In another example, the antibody or an antigen-binding fragment thereof includes a heavy chain variable region essentially consisting of the amino acid sequence of SEQ ID NO: 36 and a light chain variable region essentially consisting of the amino acid sequence of SEQ ID NO: 37.

In still another example, the antibody or an antigen-binding fragment thereof includes a heavy chain variable region consisting of the amino acid sequence of SEQ ID NO: 36 and a light chain variable region consisting of the amino acid sequence of SEQ ID NO:37.

The variable region of an antibody refers to the parts of the heavy or light chain of the antibody molecule having the amino acid sequences of CDRs and framework regions (FRs).

The term "antibody" used herein is an immunoglobulin molecule that can specifically bind to a target, such as a carbohydrate, a polynucleotide, a lipid, a polypeptide, or a protein, through at least one antigen recognition site located in a variable region of the immunoglobulin molecule. The term "antibody" used herein is used in the broadest sense, and thus may be intended to broadly include not only intact polyclonal or monoclonal antibodies, but also dimers, polymers, multispecific antibodies (e.g., bispecific antibodies), their antigen-binding fragments, antibody fragments, fusion proteins including immunoglobulin molecules with any other modified arrangement, including an antigen recognition site (e.g., a variable region), a synthetic antibody (e.g., "antibody mimetic"), and "FynomAb".

Antibodies are classified into five types: immunoglobulin (Ig) M, IgD, IgG, IgA, and IgE, which have heavy chains made from the heavy chain constant region genes µ, δ, γ, α, and ε, respectively. The light and heavy chains of an antibody are divided into a variable region whose amino acid sequence is different for each antibody and a constant region whose amino acid sequence is the same for each antibody. In the heavy chain constant region, there are CHI, hinge (H), CH2, and CH3 domains. Each domain consists of two β sheets, which are connected by an intramolecular disulfide bond.

The antibody of the present invention may be a chimeric antibody, a humanized antibody, or a human antibody.

The term "chimeric antibody" used herein refers to an antibody with a variable region derived from one type and a constant region derived from another type, for example, an antibody with a variable region sequence derived from a mouse antibody, and a constant region sequence derived from a human antibody. A method of preparing a chimeric antibody has been known in the art. For example, the document [Morrison, Science 229:1202 (1985)] may be referenced, and is incorporated herein by reference in its entirety.

The term "humanized antibody" used herein refers to an antibody in which one or more CDR sequences derived from the germline of a non-human species, for example, another mammal, such as a mouse or chicken are inserted into the framework sequence of a human immunoglobulin molecule. Additionally, the framework sequence may be remodified by, for example, a mutation method. Human Ig sequences may be referenced, for example, from the NCBI Database (Entez Gene). By using appropriate sequences, the immunogenicity of an antibody may be reduced, or the bindability, affinity, on-rate, off-rate, specificity, half-life, or any other suitable characteristics may be reduced, improved, or changed.

The term "human antibody" used herein refers to an antibody in which both the framework and CDR regions include variable regions derived from a human immunoglobulin sequence. The constant regions of antibodies are also derived from human immunoglobulin sequences.

The term "antigen-binding fragment" or "antibody fragment" not only typically includes at least some (e.g., one or more CDRs) of an antigen-binding domain or a variable region of at least a parent antibody, but also broadly includes any modified form that includes a recognition site specific for a target antigen, for example, other modified configurations of an immunoglobulin molecule with a specific antigen recognition site, such as a glycosylation variant of an antibody and an amino acid sequence variant of an antibody, covalently modified antibodies, and antigen-binding antibodies thereof. Specific examples of the antigen-binding fragments that can be used in the present invention include Fab, Fab', Fab'-SH, Fv, single-chain antibodies (scFv), F(ab')2 fragments, VL, VH, diabodies, triabodies, tetrabodies, minibody ((scFV-CH3)2), IgG ΔCH2, scFv-Fc, (scFv)2-Fc, fynomers, fynomers fused to antibodies (FynomAbs), dual-affinity re-targeting (DART), AlbudAbs, bispecific T-cell engagers (BiTEs), tandem diabodies (TandAbs), dual acting Fabs (DAFs), two-in-one antibodies, small modular immunopharmaceuticals (SMIPs), anticalins, FN3 monobodies, DARPins, affibodies, affilins, affimers, affitins, alphabodies, avimers, Im7, VLR, VNAR, Trimab, CrossMab, TRIDENT, nanobodies, binanobodies, di-sdFv, dual variable domain immunoglobulins (DVD-Igs), peptide modified antibodies (CovX-bodies), duobodies, and triomAbs, but the present invention is not limited thereto.

Specifically, the Fab fragment refers to a monovalent fragment consisting of VL, VH, CL, and CH1 domains.

The Fab' fragment is different from the Fab fragment in that it contains some residues added to the carboxy terminus of a CH1 domain including one or more cysteines from a hinge region of the antibody.

Fab'-SH refers to Fab' in which the cysteine residue of a constant domain has a free thiol group.

An F(ab')2 antibody fragment is produced as a pair of Fab' fragments via a hinge cysteine between the Fab' fragments.

Fv is a minimal antibody fragment containing a complete antigen recognition site and an antigen-binding site. This fragment is a dimer in which one heavy chain variable region and one light chain variable region are firmly and non-covalently linked. These two regions fold to form six hypervariable loops (three loops each from a heavy chain and a light chain), which provide amino acid residues for antigen binding and impart antigen-binding specificity to an antibody. However, even a single variable region has the ability to recognize and bind to an antigen, but with lower affinity than the entire binding sites.

A single-chain antibody scFv is an antibody fragment including VH and VL antibody domains linked by a single polypeptide chain. Preferably, the scFv polypeptide further includes a polypeptide linker between the VH and VL domains such that scFv can form a desired structure for antigen binding. In the present invention, the scFv polypeptide may also be referred to as an scFv antibody fragment, an antigen-binding fragment (scFv), a scFv antibody, an antibody scFv, or simply scFv.

A diabody refers to a small antibody fragment prepared by linking scFv fragments using a short linker (approximately 5 to 10 residues) between the VH and VL domains such that a pair is made in a chain, rather than between chains of the V domains to produce a divalent fragment, that is, a fragment with two antigen-binding sites. A dual-specific diabody is a heterodimer consisting of two "cross-pairing" scFv fragments present on polypeptide chains each with different VH and VL domains of two antibodies. Similarly, a triabody and a tetrabody form three antigen-binding sites and four antigen-binding sites, which include the same or different three polypeptide chains and four polypeptide chains, respectively.

A fynomer refers to a non-immunoglobulin-derived binding polypeptide derived from the human FynSH3 domain. A FynSH3-derived polypeptide is well known in the art, and described, for example, in the document [Grabulovski et al. (2007) JBC, 282, p. 3196-3204], and WO 2008/022759. A fynomer may be fused with a different molecule (e.g., an antibody) in a genetically engineered manner to produce "FynomAb", which is a form capable of being engineered to have dual specificity.

Dual-affinity re-targeting (DART) and TRIDENT refers to an antibody designed to simultaneously bind to two or more targets. DART refers to a covalently-linked dual-specific diabody, for example, a diabody linked via a C-terminus disulfide bridge, and its specific structure and definition are described in the document [J. Mol. Biol. (2010) 399, 436-449].

The antibody or an antigen-binding fragment thereof in the present invention may be named using the symbols listed in Tables 1 to 7. For example, in the present invention, the antibodies or antigen-binding fragments thereof, including the heavy chain CDR1, the heavy chain CDR2, the heavy chain CDR3, the light chain CDR1, the light chain CDR2, and the light chain CDR3, which have the amino acid sequences of SEQ ID NOs: 1, 4, 6, 2, 5, and 7, respectively, are collectively referred to as "101." In the present invention, the anti-CNTN4 antibodies or antigen-binding fragments thereof, including the heavy chain variable region and the light chain variable region, which have the amino acid sequences of SEQ ID NOs: 36 and 37, respectively, are collectively referred to as "A101." According to a specific embodiment, "A101" may refer to an antigen-binding fragment, particularly, scFv, and an antibody prepared using this may be referred to as A101-IgG antibody.

The anti-CNTN4 antibody or an antigen-binding fragment thereof may specifically bind to a CNTN4 protein, preferably, a human or mouse CNTN4 protein.

The term "specifically binding to" or "specific for" used herein means having selectivity for the presence of a target in the presence of a heterogeneous population of molecules including biological molecules, and refers to a measurable and reproducible interaction such as binding between a target and an antibody. For example, an antibody specifically binding to a particular target (e.g., an epitope) means an antibody that binds to the target with greater affinity, more readily, and/or with a longer duration than when binding to other targets.

The term "specifically binding to a CNTN4 protein" means an antibody binding to a CNTN4 protein with an equilibrium dissociation constant (KD) of 5 x 10⁻⁸ M or less, preferably, 1 x 10⁻⁸ M or less, and more preferably, 5 x 10⁻⁹ M or less. Accordingly, the anti-CNTN4 antibody or an antigen-binding fragment thereof of the present invention may bind to a CNTN4 protein, for example, a human CNTN4 protein with an equilibrium dissociation constant (KD) of 5 x 10⁻⁸ M or less, preferably, 1 x 10⁻⁸ M or less, and more preferably, 5 x 10⁻⁹ M or less.

The term "KD" used herein refers to a binding equilibrium dissociation constant (KD) of a specific antibody-antigen interaction, calculated using the formula KD = Kd/Ka (where Ka is the association rate constant and Kd is the dissociation rate constant), and the constant KD has units of M. The KD value for an antibody may be measured using a method widely established in the art. A preferable method for measuring the KD value of an antibody may be surface plasmon resonance (SRP), preferably, a biosensor system, such as the Biacore^{®} system, or bio-layer interferometry (BLI), for example, the Octet^{®} system. In one specific example, the KD described in the present invention may be a value obtained through SPR.

In one embodiment, the anti-CNTN4 antibody or an antigen-binding fragment thereof of the present invention is monospecific, and specifically binds to a single epitope, that is, a CNTN4 protein.

In another embodiment, the anti-CNTN4 antibody or an antigen-binding fragment thereof of the present invention is a multispecific, for example, bispecific or trispecific antibody molecule. A multispecific antibody molecule includes a plurality of variable regions, and each variable region has binding specificity to a different epitope. In one embodiment, the first variable region of a bispecific antibody molecule has first binding specificity to a first epitope, for example, a CNTN4 protein, and the second variable region has second binding specificity to a second epitope, for example, a target protein other than the CNTN4 protein.

In one specific example, a bispecific antibody molecule may bind to an immune checkpoint protein. An immune checkpoint protein is the generic term for proteins involved in a pathway that inhibits immune responses and proteins involved in a pathway that activates immune responses. The immune checkpoint protein may be a protein involved in a signaling pathway that inhibits the activity of regulatory T cells, or a protein involved in a signaling pathway that directly stimulates effector T cells or memory T cells. Such immune checkpoint proteins include PD-L1, PD-1, CTLA-4, BTLA, KIR, LAG3, TIM3, OX40, VISTA, TIGIT, 4-1BB, GITR, and A2aR, but the present invention is not limited thereto.

In one specific example, the bispecific antibody molecule may specifically bind to any one of PD-L1, PD-1, CTLA-4, BTLA, KIR, LAG3, TIM3, OX40, VISTA, TIGIT, 4-1BB, GITR, and A2aR; and CNTN4.

Alternatively, the bispecific antibody molecule serves as a bispecific T- cell engager, which may specifically bind to any one of CD3, 4-1BB, and CD28; and CNTN4.

Alternatively, the bispecific antibody molecule may bind to a cytokine that activates the immune system, and CNTN4. A cytokine that activates the immune system may be, for example, IFN-γ, TNF-α, IL-2, IL-6, or IL-12, but the present invention is not limited thereto. In one specific example, the bispecific antibody molecule may specifically bind to any one of IFN-γ, TNF-α, IL-2, IL-6, and IL-12; and CNTN4.

In a specific embodiment, the bispecific antibody molecule may specifically bind to CNTN4; and any one of PD-L1, PD-1, CTLA-4, BTLA, KIR, LAG3, TIM3, OX40, VISTA, TIGIT, 4-1BB, GITR, A2aR, CD3, 4-1BB, CD28, IFN-γ, TNF-α, IL-2, IL-6, and IL-12. In another specific example, any combination of the above-mentioned molecules may be prepared as a multispecific antibody molecule, for example, a trispecific antibody having first binding specificity to CNTN4, and second and third binding specificities to two types or more of CTLA-4, PD-1, and PD-L1. A multispecific antibody molecule of the present invention may be produced using standard molecular biology techniques (e.g., recombinant DNA and protein expression technologies).

In another embodiment, the antibody or an antigen-binding fragment of the present invention may which may be conjugated with a drug to form an antibody-drug conjugate (ADC). The "antibody-drug conjugate" or "ADC" used herein may be represented by the formula M-[L-(D)m]n , where M is an antibody molecule, that is, the anti-CNTN4 antibody or an antigen-binding fragment thereof of the present invention, L is an optional linker or linker unit, D is a suitable drug or prodrug, and each of m and n is an integer from approximately 1 to approximately 20. A drug included in ADC may be suitably selected depending on a therapeutic or diagnostic use, as long as it does not interfere with the specific binding of the antibody of the present invention. In one embodiment, the drug may include an adjuvant, a cytotoxic agent (e.g., a chemotherapeutic agent), a prodrug converting enzyme, a radioactive isotope or compound, or a toxin, but the present invention is not limited thereto. An adjuvant is a material for non-specifically promoting an immune response to an antigen in the early activation process of immune cells, and is referred to as an agent for reinforcing immunity by enhancing cell activity in the immune system. A drug and a linker that can be used in ADC and methods of preparing them, may follow known methods in the art. Accordingly, the present invention provides an antibody-drug conjugate including an anti-CNTN4 antibody or an antigen-binding fragment thereof.

In another embodiment, the anti-CNTN4 antibody or an antigen-binding fragment thereof of the present invention may bind to a CNTN4 protein (e.g., a human or mouse CNTN4 protein) at an EC50 of 100 nM or less, preferably, 50 nM or less, more preferably, 10 nM or less, more and more preferably, 1 nM or less, and particularly, 0.1 nM or less or 0.05 nM or less.

The term "EC50" used herein is a term associated with in vitro or in vivo analysis using an antibody, meaning the concentration of an antibody inducing 50% of the maximum response, i.e., a response halfway between the baseline and the maximum response.

### Nucleic acid molecule and vector

Another aspect of the present invention relates to a nucleic acid molecule encoding an anti-CNTN4 antibody or an antigen-binding fragment thereof of the present invention.

A nucleic acid may be present in whole cells or a cell lysate, or specifically in a purified form or a substantially pure form. The nucleic acid is an "isolated" or "substantially pure" nucleic acid that has been separated and purified from other cell components or other contaminants, such as other cell nucleic acids or proteins, by a standard technique, such as alkaline/SDS treatment, CsCl banding, column chromatography, agarose gel electrophoresis, and other methods known in the art.

The nucleic acid of the present invention may be, for example, DNA or RNA, and may contain or not contain an intron sequence. In an exemplary embodiment, the nucleic acid is a cDNA molecule.

In one embodiment, the nucleic acid molecule of the present invention encodes a heavy chain region, a light chain region, or both heavy and light chain regions, and preferably, a heavy chain variable region, a light chain variable region, or both heavy and light chain variable regions of the anti-CNTN4 antibody or an antigen-binding fragment thereof of the present invention. In one embodiment, the nucleic acid molecule of the present invention encodes a heavy chain variable region having any one amino acid sequence selected from the group consisting of SEQ ID NOs: 36, 38 to 48, 52., and 54 to 67, for example, a heavy chain variable region of SEQ ID NO: 36, and/or a light chain variable region having any one amino acid sequence selected from the group consisting of SEQ ID NOs: 37, 49 to 51, and 53, for example, a light chain variable region of SEQ ID NO: 37. Alternatively, the nucleic acid molecule of the present invention encodes the heavy chain variable regions and/or light chain variable regions of A101 to A114, and A201 to A219.

Once a DNA fragment(s) encoding the VL and/or VH regions is(are) obtained, such DNA fragment(s) may be additionally manipulated by, for example, standard recombinant DNA technology, resulting in conversion of a variable region gene to a full-length antibody chain gene, a Fab fragment gene, or an scFv gene. In this manipulation process, the VL- or VH-encoding DNA fragment operably binds to a different protein, for example, an antibody constant region, such as an hIgG1 Fc (hFc) region, an hIgG4 Fc region (e.g., hIgG4 with a S228P mutation (S228P)), or an hCK region, or another DNA fragment encoding a flexible linker. The term "operably bind/bound" used herein means that two DNA fragments are linked such that the amino acid sequences encoded by the two DNA fragments remain in-frame.

The isolated DNA encoding the VH domain may be converted to a full-length heavy chain gene by operably binding the VH-encoding DNA to another DNA molecule encoding a heavy chain constant region (CH1, H, CH2, or CH3). The heavy chain constant region may be a constant region of IgG1, IgG2, IgG3, IgG4, IgA, IgE, IgM, or IgD. In one exemplary embodiment of the present invention, the heavy chain constant region may be IgG4, and specifically, hIgG4 (S228P).

In the heavy chain gene of the Fab fragment, the VH-encoding DNA may be operably bound to another DNA molecule only encoding the constant region of the heavy chain CH1.

To produce the scFv gene, the VL- and VH-encoding DNA fragments may be operably bound to a flexible linker, for example, another fragment encoding the amino acid sequence (Gly4-Ser)3. As a result, the VL and VH sequences may be expressed as continuous single-chain proteins with VL and VH domains linked by a flexible linker.

The nucleic acid sequence of the present invention, for example, RNA or DNA, may be isolated from various sources, genetically engineered, amplified, and/or recombinantly expressed. Any recombinant expression systems including insect or mammalian systems, in addition to bacteria, e.g., yeast systems may be used. For example, nucleic acid manipulation such as subcloning into expression vectors, labeling probes, sequencing, and hybridization may be performed as known in the art.

Accordingly, the present invention provides a recombinant expression vector including the nucleic acid molecule.

The term "vector" used herein refers to a DNA molecule capable of self replication in prokaryotic and/or eukaryotic cells, is used interchangeably with a recombinant vector, a cloning vector, and an expression vector, and is generally used as an intermediate carrier for delivering a gene or a DNA fragment into cells. A vector usually includes an origin og replication that enables replication in prokaryotic and/or eukaryotic cells, a selection marker gene capable of imparting resistance to a specific condition/material such as an antibiotic degrading enzyme, a promoter enabling the transcription of a gene in eukaryotic or prokaryotic cells, and a translatable sequence, but the present invention is not limited thereto.

One type of vector is a "plasmid," which refers to a circular duplex standard DNA loop into which additional DNA fragments can be ligated. Another type of vector is a viral vector in which an additional DNA fragment can be ligated into the viral genome. Specific vectors may be autonomously replicated in host cells into which they are introduced (e.g., a bacterial vector with a bacterial origin of replication and an episomal mammalian vector).

The present invention also provides a host cell including the vector. The host cells include mammalian, plant, insect, fungal or bacterial cells, but the present invention is not limited thereto. Bacterial cells include cells derived from Gram-positive bacteria or Gram-negative bacteria, for example, some species of the genus Escherichia, such as E. coli and the genus Pseudomonas. Among the fungal cells, preferably, yeast cells may be used. Expression in yeast may be achieved using yeast strains, for example, particularly, Pichia pastoris, Saccharomyces cerevisiae, and Hansenula polymorpha. In addition, insect cells, for example, Drosophila- and Sf9-derived cells may be used as host cells. In addition, expression systems using mammalian cells, for example, Chinese hamster ovary (CHO) cells, simian COS cells, BHK cells, NSO cells, or Bowes melanoma cells may be used.

The antibody or an antigen-binding fragment thereof of the present invention may be ultimately administered to a human, and therefore a fully human expression system is particularly preferable. In this case, the host cells may be human cells, for example, HeLa, 911, AT1080, A549, 293 and HEK293 cells, and specifically, Expi293F cells.

### Preparation of antibody or antigen-binding fragment thereof

The antibody or an antigen-binding fragment thereof of the present invention may be prepared by a conventionally known method.

In one embodiment, the antibody of the present invention, for example, a monoclonal antibody, may be prepared by injecting a CNTN4 antigen into a test subject (e.g., a mouse) according to a method known in the art, and then isolating a hybridoma expressing an antibody with the desired sequence or functional characteristics.

DNA encoding the monoclonal antibody is immediately isolated and sequenced by a conventional method (for example, using oligonucleotide probes capable of specifically binding to genes that encode the heavy and light chains of the monoclonal antibody). Hybridoma cells are provided as a preferable source of this DNA. Once isolated, the DNA is placed into an expression vector, and then transfected into host cells, for example, E. coli cells, simian COS cells, CHO cells, or myeloma cells that do not otherwise produce an immunoglobulin protein to achieve the synthesis of monoclonal antibodies in recombinant host cells.

In another embodiment, the antibody or an antigen-binding fragment thereof of the present invention may be prepared by using antibody display technology, for example, phage library technology.

An antibody phage library is constructed, for example, by cloning the variable region genes of the heavy and light chains of a human antibody fused to a phage coat protein (plll) within a phagemid vector to express them in E. coli, and infecting them with an M13 helper phage, thereby displaying antibody fragments (scFv or Fab) with diverse combinations of heavy and light chain variable region sequences on the surface of the phage. A specific human monoclonal antibody is produced by isolating a fragment of an antibody binding to a specific antigen from this library using a panning method, characterizing the separated antibody fragment, and converting it into a whole IgG form and overexpressing it in animal cells.

The term "phagemid vector" used herein is plasmid DNA with a phage origin of replication, and typically has an antibiotic-resistant gene as a selection marker. A phagemid vector used in phage display includes the gIII gene of the M13 phage or a part thereof, and the scFv gene is ligated at the 5' end of the gIII gene and expressed through a transformant.

A "helper phage" is a phage providing genetic information needed for the phagemid to be assembled into phage particles. Since the phagemid includes gIII or only a part of the phage genes, host cells (transformants) transformed with the phagemid are infected with helper phages to provide other phage genes. Helper phages include M13K07 and VCSM13, and mostly, they have a gene resistant to an antibiotic, such as kanamycin, so a transformant infected with a helper phage can be selected. In addition, due to a defective packaging signal, a phagemid gene is selectively assembled into a phage particle rather than a helper phage gene.

### Uses and methods

The antibody or an antigen-binding fragment thereof of the present invention restores an immune response suppressed by the binding of the CNTN4 protein. It has been confirmed that the CNTN4 protein inhibits the proliferation of T cells, particularly, CD4+ T cells and CD8+T cells. Accordingly, the antibody or an antigen-binding fragment thereof of the present invention may specifically bind to the CNTN4 protein, thereby enhancing the activity of T cells, particularly, CD4+ T cells or CD8+ T cells, and thus it can be used in treatment of a disease associated with immunosuppression.

In one embodiment, the antibody or an antigen-binding fragment thereof of the present invention enhances T cell activity. Accordingly, it can be seen that the antibody or an antigen-binding fragment thereof of the present invention activates T cells, particularly, CD4+ T cells or CD8+ T cells. In one specific example, the antibody or an antigen-binding fragment thereof of the present invention may enhance the proliferation of T cells suppressed by CNTN4. The antibody or an antigen-binding fragment thereof of the present invention exhibits excellent CNTN4 neutralization ability.

Accordingly, the present invention relates to induction of T cell activation using the anti-CNTN4 antibody or an antigen-binding fragment thereof. In one embodiment, the present invention provides a method of inducing or enhancing T cell activation, which includes administering an effective amount of the anti-CNTN4 antibody or an antigen-binding fragment thereof to a subject. In yet another embodiment, the present invention provides a use of the anti-CNTN4 antibody or an antigen-binding fragment thereof for inducing or enhancing T cell activation. In yet another embodiment, the present invention provides a pharmaceutical composition for inducing or enhancing T cell activation, which includes the anti-CNTN4 antibody or an antigen-binding fragment thereof.

Accordingly, the present invention relates to prevention, improvement, or treatment of an immunosuppression-associated disease using the anti-CNTN4 antibody or an antigen-binding fragment thereof.

The term "prevention" used herein refers to all actions inhibiting an immunosuppression-related disease, or delaying the development of an immunosuppression-associated disease, and "treatment" used herein refers to all actions involved in alleviating or beneficially changing the symptoms of an immunosuppression-associated disease.

In one embodiment, the present invention provides a method of preventing, improving or treating an immunosuppression-associated disease, which includes administering an effective amount of the anti-CNTN4 antibody or an antigen-binding fragment thereof to a subject.

In another embodiment, the present invention provides a method of preventing or treating cancer in a subject, which includes administering the anti-CNTN4 antibody or an antigen-binding fragment thereof to the subject.

In another embodiment, the present invention provides a use of an anti-CNTN4 antibody or an antigen-binding fragment thereof for preventing, improving or treating an immunosuppression-associated disease.

In another embodiment, the present invention provides a use of the anti-CNTN4 antibody or an antigen-binding fragment thereof for preparing a drug for preventing or treating cancer.

In another embodiment, the present invention provides a pharmaceutical composition for preventing, improving or treating an immunosuppression-associated disease, including the anti-CNTN4 antibody or an antigen-binding fragment thereof.

The term "subject" used herein means both a human and a non-human animal. Non-human animals include all vertebrates, e.g., mammals and non-mammals, such as primates other than humans, sheep, dogs, cats, cows, horses, chickens, amphibians and reptiles, and preferably, mammals such as primates other than humans, sheep, dogs, cats, cows, and horses. A preferable subject is a human who requires the activation or enhancement of an immune response.

Preferably, the anti-CNTN4 antibody or an antigen-binding fragment thereof of the present invention may activate T cells by blocking the binding of a CNTN4 protein, and/or enhance an immune response to cancer cells in a cancer patient, thereby inhibiting the growth of cancer cells in vivo, and thus it can be useful for the prevention, improvement or treatment of cancer.

Preferred cancers whose growth can be inhibited using the antibody of the present invention typically include cancers that typically respond to immunotherapy. For example, the cancer of the present invention includes melanoma (e.g., metastatic melanoma), kidney cancer (e.g., clear cell carcinoma), prostate cancer (e.g., hormone-refractory prostatic adenocarcinoma), breast cancer, colorectal cancer, rectal cancer, colon cancer, and lung cancer (e.g., non-small cell lung cancer), but the present invention is not limited thereto. In addition, a target to be treated in the present invention includes refractory or recurrent malignant tumors whose growth can be inhibited using the antibody of the present invention.

Examples of other cancers to be treated using the method of the present invention include gallbladder cancer, liver cancer, bone cancer, pancreatic cancer, skin cancer, head and neck cancers, malignant melanoma of the skin and intraocular melanoma, uterine cancer, ovarian cancer, rectal cancer, anal cancer, stomach cancer, testicular cancer, uterine cancer, fallopian tube carcinoma, endometrial carcinoma, cervical carcinoma, vaginal carcinoma, vulvar carcinoma, Hodgkin's lymphoma, non-Hodgkin's lymphoma, esophageal cancer, small intestine cancer, endocrine system cancers, thyroid cancer, parathyroid cancer, adrenal cancer, sarcomas such as osteosarcoma or soft tissue sarcoma (e.g., Kaposi's sarcoma), urethral cancer, penile cancer, chronic and acute leukemias including acute myeloid leukemia, chronic myeloid leukemia, acute lymphoblastic leukemia, chronic lymphocytic leukemia, pediatric solid tumors, lymphocytic lymphoma, bladder cancer, ureteral cancer, renal pelvis carcinoma, neoplasms of the central nervous system (CNS), primary CNS lymphoma, tumor angiogenesis, spinal cord tumors, brainstem glioma, pituitary adenoma, basal cell carcinoma, squamous cell carcinoma, T-cell lymphoma, environmentally induced cancers such as asbestos-related cancers, and a combination thereof.

In another embodiment, the cancer may be cancer expressing CNTN4.

In another embodiment, the cancer may be refractory or resistant to an existing immune checkpoint inhibitor (e. g., resistant to a PD-1 pathway inhibitor, a PD-L1 pathway inhibitor, or a CTLA-4 pathway inhibitor).

The antibody or an antigen-binding fragment of the present invention may be used alone or in combination with another anticancer therapy. The other anticancer therapy may be, for example, a standard cancer therapy (e.g., chemotherapy, radiation therapy, or surgery); or other anticancer agents, for example, a cytotoxic agent, a cell proliferation inhibitor, an anti-angiogenic or anti-metabolite agent, a tumor-targeting agent, an immune stimulator or immunomodulator, or an antibody conjugated to a cytotoxic agent, a cell proliferation inhibitor, or another toxicant, or an immune checkpoint inhibitor.

Preferably, the antibody or an antigen-binding fragment of the present invention may be used in combination with another anticancer agent, such as an immune checkpoint inhibitor, a chemotherapeutic agent, or radiation therapy. The immune checkpoint inhibitor may be, for example, an anti-CTLA-4 antibody (e.g., ipilimumab), an anti-PD-1 antibody (e.g., pembrolizumab or nivolumab), or an anti-PD-L1 antibody (e.g., atezolizumab, avelumab, or durvalumab). The chemotherapeutic agent may include an alkylating agent, an antimetabolite, a kinase inhibitor, a spindle toxic plant alkaloid, a cytotoxic/antitumor antibiotic, a topoisomerase inhibitor, a photosensitizer, an antiestrogen, and a selective estrogen receptor modulator (SERM), an antiprogesterone, an estrogen receptor downregulator (ERD), an estrogen receptor antagonist, a luteinizing hormone-releasing hormone agonist, an antiandrogen, an aromatase inhibitor, an EGFR inhibitor, a VEGF inhibitor, and an antisense oligonucleotide that inhibits the expression of a gene involved in abnormal cell proliferation or tumor growth, but the present invention is not limited thereto. Specific examples of the chemotherapeutic agents of the present invention include gemcitabine, vinorelbine, etoposide (VP-16), a platinum analog such as cisplatin or carboplatin, and a taxoid such as paclitaxel, albumin-bound paclitaxel, or docetaxel.

In the case of co-administration with another anticancer agent, the antibody or an antigen-binding fragment of the present invention may be administered separately or applied in the form of combined product in which a plurality of active components are present in one pharmaceutical preparation. In the case of separate administration, the two preparations may be sequentially or simultaneously administered. In the case of simultaneous administration, the two preparations are administered together to a patient. In the case of sequential administration, these two preparations may be administered with a brief time difference, for example, administered to a patient within 12 hours or less, or 6 hours or less.

In one embodiment, the present invention provides a method of preventing, improving or treating an immunosuppression-associated disease, for example, cancer, which includes administering an effective amount of the anti-CNTN4 antibody or an antigen-binding fragment thereof in combination with an additional anticancer agent to a subject. This method includes simultaneously or sequentially administering compositions separately including the antibody and the anticancer agent, as well as simultaneously administering the anti-CNTN4 antibody or an antigen-binding fragment thereof in combination with the additional anticancer agent in one composition to a patient in need thereof.

In another embodiment, the present invention provides a use of the anti-CNTN4 antibody or an antigen-binding fragment thereof in combination with an additional anticancer agent for preventing, improving or treating an immunosuppression-associated disease, for example, cancer.

In another embodiment, the present invention provides a pharmaceutical composition or composite for preventing, improving or treating an immunosuppression-associated disease, for example, cancer, which includes the anti-CNTN4 antibody or an antigen-binding fragment thereof, and an additional anticancer agent. In the present invention, the pharmaceutical composition or composite, which includes the anti-CNTN4 antibody or an antigen-binding fragment thereof, and an additional anticancer agent, includes not only cases in which both components are physically present in one preparation but also cases in which both components are administered simultaneously or sequentially in separate preparations. Here, two drugs may be individually provided or provided in one kit. Accordingly, the present invention provides a kit for preventing, improving or treating an immunosuppression-associated disease, for example, cancer, which includes the anti-CNTN4 antibody or an antigen-binding fragment thereof, and an additional anticancer agent.

The additional anticancer agent is preferably an immune checkpoint inhibitor, more preferably, an anti-CTLA-4 antibody (e.g., ipilimumab), an anti-PD-1 antibody (e.g., pembrolizumab or nivolumab), or an anti-PD-L1 antibody (e.g., atezolizumab, avelumab, or durvalumab).

Another preferable additional anticancer agent may be a chemotherapeutic agent, for example, gemcitabine, vinorelbine, etoposide (VP-16), a platinum analog such as cisplatin or carboplatin, or a taxoid such as paclitaxel, albumin-bound paclitaxel, or docetaxel.

Another preferable additional anticancer agent used in combination with the antibody or an antigen-binding fragment thereof of the present invention may be radiation therapy.

The present invention also provides a method of detecting the presence of the CNTN4 protein in a sample or measuring the amount of the anti-CNTN4 antibody using the anti-CNTN4 antibody or an antigen-binding fragment thereof as an active ingredient. The method includes bringing the antibody or an antigen-binding fragment thereof into contact with a sample and a control sample under conditions that can form a complex by binding the antibody or an antigen-binding fragment thereof to the CNTN4 protein. Afterward, it is detected whether the complex is formed, and here, a difference in the degree of complex formation between samples, compared to the control sample, is evidence of the presence of human blood antigens in the sample (e.g., blood).

Accordingly, the present invention provides a composition for diagnosing cancer, which includes the anti-CNTN4 antibody or an antigen-binding fragment thereof.

### Pharmaceutical composition

The present invention provides a pharmaceutical composition, which includes the anti-CNTN4 antibody or an antigen-binding fragment thereof. The composition may include an inactive ingredient, i.e., a pharmaceutically acceptable excipient [refer to Handbook of Pharmaceutical Excipients, etc.]. A composition of therapeutic and diagnostic agents may be prepared by mixing physiologically acceptable carriers, excipients or stabilizers in the form of a freeze-dried powder, a slurry, an aqueous solution or a suspension.

Suitable administration routes include parenteral administration, for example, intramuscular, intravenous, or subcutaneous administration. The administration of the antibody used in the pharmaceutical composition of the present invention or used to perform the method of the present invention may be carried out by a variety of conventional methods, including topical application, or percutaneous, subcutaneous, intraperitoneal, parenteral, intraarterial or intravenous injection. In one embodiment, the antibody of the present invention is administered intravenously or subcutaneously.

Hereinafter, the present invention will be described in further detail through examples. The following examples are merely provided to more specifically describe the present invention, and it will be obvious to those of ordinary skill in the art that the scope of the present invention is not limited to the examples according to the gist of the present invention.

### Modes of the Invention

### Example 1: Preparation of CNTN4-specifically-binding scFv and confirmation of its binding affinity (1)

### 1.1 Construction of scFv library (1)

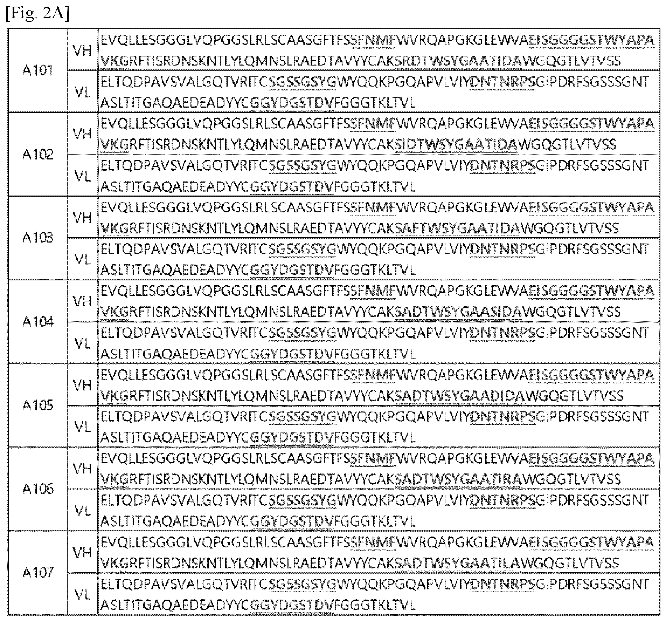

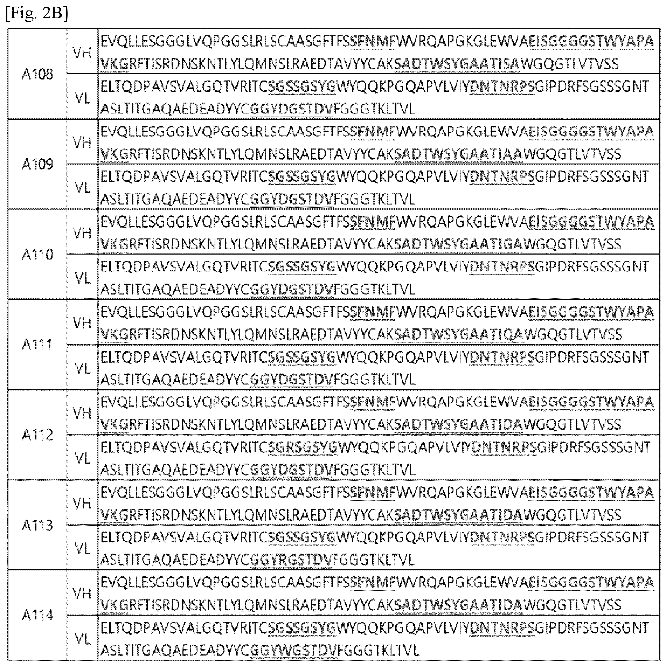

A total of 14 scFv fragments were obtained by electroporating the purified PCR products for the CDRs in Table 1 into E. coli BL21, the amino acid sequences of the heavy and light chain variable regions of scFvs are shown in FIG. 2 and Table 2 (The underlined and bolded sequences in FIG. 2 indicate CDR sequences in each region).

**[Table 1]**

| No. | | CDR-1 | | CDR-2 | | CDR-3 | |
|---|---|---|---|---|---|---|---|
| 101 | VH | SFNMF | SEQ ID NO: 1 | EISGGGGSTWYAPA VKG | SEQ ID NO: 4 | SRDTWSYGAATIDA | SEQ ID NO: 6 |
| | VL | SGSSGSYG | SEQ ID NO: 2 | DNTNRPS | SEQ ID NO: 5 | GGYDGSTDV | SEQ ID NO: 7 |
| 102 | VH | SFNMF | SEQ ID NO: 1 | EISGGGGSTWYAPA VKG | SEQ ID NO: 4 | SIDTWSYGAATIDA | SEQ ID NO: 8 |
| | VL | SGSSGSYG | SEQ ID NO: 2 | DNTNRPS | SEQ ID NO: 5 | GGYDGSTDV | SEQ ID NO: 7 |
| 103 | VH | SFNMF | SEQ ID NO: 1 | EISGGGGSTWYAPA VKG | SEQ ID NO: 4 | SAFTWSYGAATIDA | SEQ ID NO: 9 |
| | VL | SGSSGSYG | SEQ ID NO: 2 | DNTNRPS | SEQ ID NO: 5 | GGYDGSTDV | SEQ ID NO: 7 |
| 104 | VH | SFNMF | SEQ ID NO: 1 | EISGGGGSTWYAPA VKG | SEQ ID NO: 4 | SADTWSYGAASIDA | SEQ ID NO: 10 |
| | VL | SGSSGSYG | SEQ ID NO: 2 | DNTNRPS | SEQ ID NO: 5 | GGYDGSTDV | SEQ ID NO: 7 |
| 105 | VH | SFNMF | SEQ ID NO: 1 | EISGGGGSTWYAPA VKG | SEQ ID NO: 4 | SADTWSYGAADIDA | SEQ ID NO:11 |
| | VL | SGSSGSYG | SEQ ID NO: 2 | DNTNRPS | SEQ ID NO: 5 | GGYDGSTDV | SEQ ID NO: 7 |
| 106 | VH | SFNMF | SEQ ID NO: 1 | EISGGGGSTWYAPA VKG | SEQ ID NO: 4 | SADTWSYGAATIRA | SEQ ID NO: 12 |
| | VL | SGSSGSYG | SEQ ID NO: 2 | DNTNRPS | SEQ ID NO: 5 | GGYDGSTDV | SEQ ID NO: 7 |
| 107 | VH | SFNMF | SEQ ID NO: 1 | EISGGGGSTWYAPA VKG | SEQ ID NO: 4 | SADTWSYGAATILA | SEQ ID NO:13 |
| | VL | SGSSGSYG | SEQ ID NO: 2 | DNTNRPS | SEQ ID NO: 5 | GGYDGSTDV | SEQ ID NO: 7 |
| 108 | VH | SFNMF | SEQ ID NO: 1 | EISGGGGSTWYAPA VKG | SEQ ID NO: 4 | SADTWSYGAATISA | SEQ ID NO:14 |
| | VL | SGSSGSYG | SEQ ID NO: 2 | DNTNRPS | SEQ ID NO: 5 | GGYDGSTDV | SEQ ID NO: 7 |
| 109 | VH | SFNMF | SEQ ID NO: 1 | EISGGGGSTWYAPA VKG | SEQ ID NO: 4 | SADTWSYGAATIAA | SEQ ID NO:15 |
| | VL | SGSSGSYG | SEQ ID NO: 2 | DNTNRPS | SEQ ID NO: 5 | GGYDGSTDV | SEQ ID NO: 7 |
| 110 | VH | SFNMF | SEQ ID NO: 1 | EISGGGGSTWYAPA VKG | SEQ ID NO: 4 | SADTWSYGAATIGA | SEQ ID NO: 16 |
| | VL | SGSSGSYG | SEQ ID NO: 2 | DNTNRPS | SEQ ID NO: 5 | GGYDGSTDV | SEQ ID NO: 7 |
| 111 | VII | SFNMF | SEQ ID NO: 1 | EISGGGGSTWYAPA VKG | SEQ ID NO: 4 | SADTWSYGAATIQA | SEQ ID NO: 17 |
| | VL | SGSSGSYG | SEQ ID NO: 2 | DNTNRPS | SEQ ID NO: 5 | GGYDGSTDV | SEQ ID NO: 7 |
| 112 | VH | SFNMF | SEQ ID NO: 1 | EISGGGGSTWYAPA VKG | SEQ ID NO: 4 | SADTWSYGAATIDA | SEQ ID NO:18 |
| | VL | SGRSGSYG | SEQ ID NO: 3 | DNTNRPS | SEQ ID NO: 5 | GGYDGSTDV | SEQ ID NO: 7 |
| 113 | VH | SFNMF | SEQ ID NO: 1 | EISGGGGSTWYAPA VKG | SEQ ID NO: 4 | SADTWSYGAATIDA | SEQ ID NO:18 |
| | VL | SGSSGSYG | SEQ ID NO: 2 | DNTNRPS | SEQ ID NO: 5 | GGYRGSTDV | SEQ ID NO:19 |
| 114 | VH | SFNMF | SEQ ID NO: 1 | EISGGGGSTWYAPA VKG | SEQ ID NO: 4 | SADTWSYGAATIDA | SEQ ID NO:18 |
| | VL | SGSSGSYG | SEQ ID NO: 2 | DNTNRPS | SEQ ID NO: 5 | GGYWGSTDV | SEQ ID NO: 20 |

**[Table 2]**

| | | | |
|---|---|---|---|
| A101 | VH | SEQ ID NO: 36 | EVQLLESGGGLVQPGGSLRLSCAASGFTFSSFNMFWVRQAPGKGLEWVAEISGGG GSTWYAPAVKGRFTISRDNSKNTLYLQMNSLRAEDTAVYYCAKSRDTWSYGAAT IDAWGQGTLVTVSS |
| | VL | SEQ ID NO: 37 | ELTQDPAVSVALGQTVRITCSGSSGSYGWYQQKPGQAPVLVIYDNTNRPSGIPDRF SGSSSGNTASLTITGAQAEDEADYYCGGYDGSTDVFGGGTKLTVL |
| A102 | VH | SEQ ID NO: 38 | EVQLLESGGGLVQPGGSLRLSCAASGFTFSSFNMFVWRQAPGKGLEVVWAEISGGG GSTWYAPAVKGRFTISRDNSKNTLYLQMNSLRAEDTAVYYCAKSIDTWSYGAATI DAWGQGTLVTVSS |
| | VL | SEQ ID NO: 37 | ELTQDPAVSVALGQTVRITCSGSSGSYGWYQQKPGQAPVLVIYDNTNRPSGIPDRF SGSSSGNTASLTITGAQAEDEADYYCGGYDGSTDVFGGGTKLTVL |
| A103 | VH | SEQ ID NO: 39 | EVQLLESGGGLVQPGGSLRLSCAASGFTFSSFNMFWVRQAPGKGLEWVAEISGGG GSTWYAPAVKGRFTISRDNSKNTLYLQMNSLRAEDTAVYYCAKSAFTWSYGAAT IDAWGQGTLVTVSS |
| | VL | SEQ ID NO: 37 | ELTQDPAVSVALGQTVRITCSGSSGSYGWYQQKPGQAPVLVIYDNTNRPSGIPDRF SGSSSGNTASLTITGAQAEDEADYYCGGYDGSTDVFGGGTKLTVL |
| A104 | VH | SEQ ID NO: 40 | EVQLLESGGGLVQPGGSLRLSCAASGFTFSSFNMFVWRQAPGKGLEVWAEISGGG GSTWYAPAVKGRFTISRDNSKNTLYLQMNSLRAEDTAVYYCAKSADTWSYGAAS IDAWGQGTLVTVSS |
| | VL | SEQ ID NO: 37 | ELTQDPAVSVALGQTVRITCSGSSGSYGWYQQKPGQAPVLVIYDNTNRPSGIPDRF SGSSSGNTASLTITGAQAEDEADYYCGGYDGSTDVFGGGTKLTVL |
| A105 | VH | SEQ ID NO: 41 | EVQLLESGGGLVQPGGSLRLSCAASGFTFSSFNMFWVRQAPGKGLEWVAEISGGG GSTWYAPAVKGRFTISRDNSKNTLYLQMNSLRAEDTAVYYCAKSADTWSYGAA DIDAWGQGTLVTVSS |
| | VL | SEQ ID NO: 37 | ELTQDPAVSVALGQTVRITCSGSSGSYGWYQQKPGQAPVLVIYDNTNRPSGIPDRF SGSSSGNT ASLTITGAQAEDEADYYCGGYDGSTDVFGGGTKLTVL |
| A106 | VH | SEQ ID NO: 42 | EVQLLESGGGLVQPGGSLRLSCAASGFTFSSFNMFWVRQAPGKGLEWVAEISGGG GSTWYAPAVKGRFTISRDNSKNTLYLQMNSLRAEDTAVYYCAKSADTWSYGAA TIRAWGQGTLVTVSS |
| | VL | SEQ ID NO: 37 | ELTQDPAVSVALGQTVRITCSGSSGSYGWYQQKPGQAPVLVIYDNTNRPSGIPDRF SGSSSGNT ASLTITGAQAEDEADYYCGGYDGSTDVFGGGTKLTVL |
| A107 | VH | SEQ ID NO: 43 | EVQLLESGGGLVQPGGSLRLSCAASGFTFSSFNMFVWRQAPGKGLEVVWAEISGGG GSTWYAPA VKGRFTISRDNSKNTLYLQMNSLRAEDTAVYYCAKSADTWSYGAATILAWGQGT LVTVSS |
| | VL | SEQ ID NO: 37 | ELTQDPAVSVALGQTVRITCSGSSGSYGWYQQKPGQAPVLVIYDNTNRPSGIPDRF SGSSSGNTASLTITGAQAEDEADYYCGGYDGSTDVFGGGTKLTVL |
| A108 | VH | SEQ ID NO: 44 | EVQLLESGGGLVQPGGSLRLSCAASGFTFSSFNMFWVRQAPGKGLEWVAEISGGG GSTWYAPAVKGRFTISRDNSKNTLYLQMNSLRAEDTAVYYCAKSADTWSYGAA TISAWGQGTLVTVSS |
| | VL | SEQ ID NO: 37 | ELTQDPAVSVALGQTVRITCSGSSGSYGWYQQKPGQAPVLVIYDNTNRPSGIPDRF SGSSSGNT ASLTITGAQAEDEADYYCGGYDGSTDVFGGGTKLTVL |
| A109 | VH | SEQ ID NO: 45 | EVQLLESGGGLVQPGGSLRLSCAASGFTFSSFNMFWVRQAPGKGLEWVAEISGGG GSTWYAPAVKGRFTISRDNSKNTLYLQMNSLRAEDTAVYYCAKSADTWSYGAA TIAAWGQGTLVTVSS |
| | VL | SEQ ID NO: 37 | ELTQDPAVSVALGQTVRITCSGSSGSYGWYQQKPGQAPVLVIYDNTNRPSGIPDRF SGSSSGNTASLTITGAQAEDEADYYCGGYDGSTDVFGGGTKLTVL |
| A110 | VH | SEQ ID NO: 46 | EVQLLESGGGLVQPGGSLRLSCAASGFTFSSFNMFWVRQAPGKGLEWVASGGGG STWYAPAVKGRFTISRDNSKNTLYLQMNSLRAEDTAVYYCAKSADTWSYGAATI GAWGQGTLVTVSS |
| | VL | SEQ ID NO: 37 | ELTQDPAVSVALGQTVRITCSGSSGSYGWYQQKPGQAPVLVIYDNTNRPSGIPDRF SGSSSGNT ASLTITGAQAEDEADYYCGGYDGSTDVFGGGTKLTVL |
| A111 | VH | SEQ ID NO: 47 | EVQLLESGGGLVQPGGSLRLSCAASGFTFSSFNMFWVRQAPGKGLEWVAEISGGG GSTWYAPAVKGRFTISRDNSKNTLYLQMNSLRAEDTAVYYCAKSADTWSYGAA TIQAWGQGTLVTVSS |
| | VL | SEQ ID NO: 37 | ELTQDPAVSVALGQTVRITCSGSGSYGWYQQKPGQAPVLVIYDNINRPSGIPDRFS GSSSGNT ASLTITGAQAEDEADYYCGGYDGSTDVFGGGTKLTVL |
| A112 | VH | SEQ ID NO: 48 | EVQLLESGGGLVQPGGSLRLSCAASGFTFSSFNMFWVRQAPGKGLEWVAEISGGG GSTWYAPAVKGRFTISRDNSKNTLYLQMNSLRAEDTAVYYCAKSADTWSYGAA TIDAWGQGTLVTVSS |
| | VL | SEQ ID NO: 49 | ELTQDPAVSVALGQTVRITCSGRSGSYGWYQQKPGQAPVLVIYDNTNRPSGIPDRF SGSSSGNTASLTITGAQAEDEADYYCGGYDGSTDVFGGGTKLTVL |
| A113 | VH | SEQ ID NO: 48 | EVQLLESGGGLVQPGGSLRLSCAASGFTFSSFNMFWVRQAPGKGLEWVASGGGG STWYAPAVKGRFTISRDNSKNTLYLQMNSLRAEDTAVYYCAKSADTWSYGAATI DAWGQGTLVTVSS |
| | VL | SEQ ID NO: 50 | ELTQDPAVSVALGQTVRITCSGSSGSYGWYQQKPGQAPVLVIYDNTNRPSGIPDRF SGSSSGNT ASLTITGAQAEDEADYYCGGYRGSTDVFGGGTKLTVL |
| A114 | VH | SEQ ID NO: 48 | EVQLLESGGGLVQPGGSLRLSCAASGFTFSSFNMFWVRQAPGKGLEWVAEISGGG GSTWYAPAVKGRFTISRDNSKNTLYLQMNSLRAEDTAVYYCAKSADTWSYGAA TIDAWGQGTLVTVSS |
| | VL | SEQ ID NO: 51 | ELTQDPAVSVALGQTVRITCSGSSGSYGWYQQKPGQAPVLVIYDNTNRPSGIPDRF SGSSSGNT ASLTITGAQAEDEADYYCGGYWGSTDVFGGGTKLTVL |

### 1.2 Confirmation of CNTN4 binding affinity of scFv (1)

### Confirmation of binding affinity of scFv to antigen protein through FACS

FACS analysis was performed on the scFvs prepared in Example 1.1.

A periplasm containing various concentrations of scFvs was incubated with mouse antigen-overexpressing 293F cells at 4 °C for 1 hour. After washing, a PE-labeled anti-His-tagged antibody was added to the wells, and incubated in the dark at 4 °C for 0.5 hours. Blank 293F cells were used as a negative control (NC). The mean fluorescence intensity (MFI) of the cells was measured by flow cytometry.

The results are shown in Table 3. It was confirmed that all of the scFvs in Example 1.1 exhibited excellent cell binding affinity to the CNTN4 protein at the nM level.

**[Table 3]**

| Sample | FACS | |
|---|---|---|
| | Mouse CNTN4 cell | |
| | Top MFI | EC50 (nM) |
| A101 | 2070 | 33.2 |
| A102 | 2110 | 17.3 |
| A103 | 2736 | 12.0 |
| A104 | 2049 | 29.4 |
| A105 | 2459 | 13.9 |
| A106 | 2736 | 72.6 |
| A107 | 4111 | 13.6 |
| A108 | 2836 | 20.2 |
| A109 | 2827 | 8.3 |
| A110 | 2154 | 20.1 |
| A111 | 4193 | 10.4 |
| A112 | 1861 | 32.5 |
| A113 | 1104 | 45.8 |
| A114 | 2261 | 7.6 |
| NC | N/A | N/A |

### Example 2: Preparation of anti-CNTN4 IgG antibody (1)

Nine (A102, A103, A104, A105, A106, A108, A109, A112, and A114) scFVs among the scFvs prepared in Example 1.1 were selected to prepare antibodies.

Specifically, the VH genes of the selected nine scFvs were fused with the CH1, hinge, CH2 and CH3 segments of human IgG4 (S228P) of a heavy chain, and the VL genes were fused with the human Ig domain of a light chain. Plasmids having heavy and light chains were co-transfected into ExpiCHO-S cells. The cells were cultured for 8 days, and the supernatant was collected. The supernatant of the cell culture was applied to a column through a sample inlet to purify a protein, and particularly, a Protein A column equilibrated with a wash buffer (50 mM Tris, 150 mM NaCl, pH 7.4) was used. After washing the column with 5 CV of the wash buffer, gradient elution was performed using buffer B (0.1 M glycine, pH 3.4). Afterward, after applying the eluted protein to a cation exchange resin, the final protein was purified and eluted using a NaCl concentration gradient in sodium acetate buffer (pH 5.0). The final eluted protein concentration was determined by A280/extinction coefficient using Nanodrop 2000. By analyzing the purified protein through SDS-PAGE and HPLC-SEC, It was confirmed that an anti-CNTN4 antibody was prepared (Table 4).

The prepared antibody was named scFv-IgG antibody (e.g., the antibody prepared using A102 scFv was named A102-IgG antibody).

**[Table 4]**

| Antibody | Concentration (mg/mL) | HPLC-SEC Purity(%) |
|---|---|---|
| A102-IgG Ab | 7.98 | 96.7 |
| A103-IgG Ab | 6.76 | 96.8 |
| A104-IgG Ab | 6.89 | 98.7 |
| A105-IgG Ab | 9.40 | 96.5 |
| A106-IgG Ab | 3.28 | 90.8 |
| A108-IgG Ab | 11.40 | 96.7 |
| A109-IgG Ab | 6.85 | 98.4 |
| A112-IgG Ab | 4.18 | 96.7 |
| A114-IgG Ab | 13.3 | 98.1 |

### Example 3: Confirmation of CNTN4 binding affinity of anti-CNTN4 IgG antibody (1)

### 3.1 ELISA test

An ELISA test was performed on the nine IgG antibodies (A102, A103, A104, A105, A106, A108, A109, A112, and A114) prepared in Example 2. In addition, a human IgG4 antibody was used as a negative control (NC). A 96-well plate was coated with anti-His antibody in a coating buffer at 4 °C overnight. Next day, the plate was blocked with 5% skim milk in PBS at 25 °C for 1 hour. Human CNTN4 (His-tagged) was added to the plate and incubated at 25 °C for 1 hour. After washing, antibodies were added to the wells and incubated at 25 °C for 2 hours. Subsequently, the plate was incubated at 25 °C for 1 hour, together with an anti-human IgG (HRP) conjugate. HRP activity was detected with a tetramethylbenzidine (TMB) substrate, and the reaction was quenched with 2M HCl. The plate was read at 450 nm.

As shown in Table 5, the results showed that all of the nine antibodies exhibited very high binding affinity, with EC50 values at the pM level.

**[Table 5]**

| Antibody | EC50 (nM) |
|---|---|
| A102-IgG Ab | 0.057 |
| A103-IgG Ab | 0.031 |
| A104-IgG Ab | 0.069 |
| A105-IgG Ab | 0.153 |
| A106-IgG Ab | 0.042 |
| A108-IgG Ab | 0.034 |
| A109-IgG Ab | 0.041 |
| A112-IgG Ab | 0.075 |
| A114-IgG Ab | 0.129 |
| NC (hIgG4) | NA |

### 3.2 FACS analysis

FACS analysis was performed on the nine IgG antibodies (A102, A103, A104, A105, A106, A108, A109, A112, and A114) prepared in Example 2. In addition, a human IgG4 antibody was used as a negative control (NC).

A HEK293 cell line that overexpresses human CNTN4 on the cell surface was manufactured. After seeding the overexpressing HEK293 cells at 1 x 105 cells/well in each well of a 96-well plate, the nine IgG antibodies pre-diluted to different concentrations were prepared and added to each well containing the cells. The highest concentration of the antibodies was set to 100 nM, and serially diluted 3-fold to 10 points. After adding the antibodies, the cells were incubated at 4 °C for 1 hour. After washing the cells twice with a FACS buffer, an APC-conjugated anti-human IgG antibody was added to the cells, and the resulting cells were further incubated at 4 °C for 30 minutes. The cells were washed twice, and 7-AAD was added to each sample at a concentration of 0.5 mg/mL. Each sample was transferred to a FACS tube, and then the binding degrees of the nine IgG antibodies were analyzed using an FACS Canto II flow cytometer.

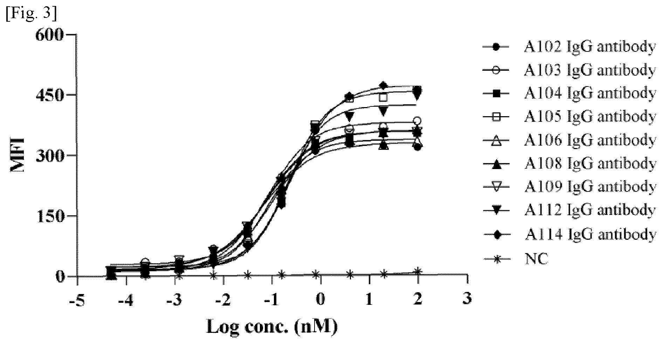

The results are shown in FIG. 3 and Table 6. It was observed that the mean fluorescence intensities (MFI) of the nine IgG antibodies were increased in a concentration-dependent manner (FIG. 3), and it was confirmed that the nine IgG antibodies exhibited high binding affinity to CNTN4 expressed on the cell surface, with the EC50 values at the pM level (Table 6).

**[Table 6]**

| Antibody | TOP MFI | EC50 (nM) |
|---|---|---|
| A102-IgG Ab | 327.6 | 0.066 |
| A103-IgG Ab | 378.5 | 0.090 |
| A104-IgG Ab | 357.6 | 0.109 |
| A105-IgG Ab | 454.2 | 0.234 |
| A106-IgG Ab | 335.7 | 0.086 |
| A108-IgG Ab | 355.9 | 0.074 |
| A109-IgG Ab | 357.1 | 0.082 |
| A112-IgG Ab | 421.5 | 0.204 |
| A114-IgG Ab | 469.9 | 0.262 |
| NC (hIgG4) | NA | NA |

### Example 4: Preparation of CNTN4-specifically-binding scFv and confirmation of its binding affinity (2)

### 4.1 Construction of scFv library (2)

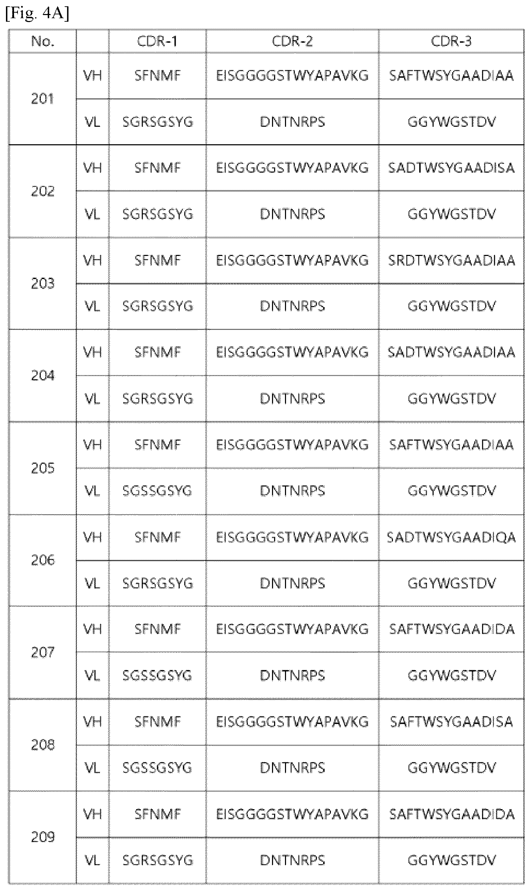

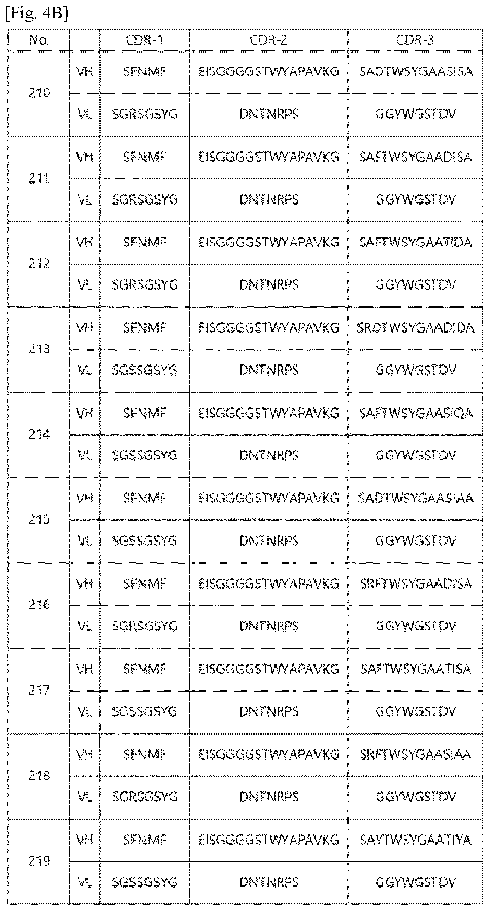

The CDR sequences of the scFvs selected in Example 1 were additionally mutated. To remove scFv post-translational modification sites, fixed mutations were introduced to create a set of mutated CDRs, as shown in FIG. 4 and Table 7.

**[Table 7]**

| No. | | CDR-1 | | CDR-2 | | CDR-3 | |
|---|---|---|---|---|---|---|---|
| 201 | VH | SFNMF | SEQ ID NO: 1 | EISGGGGSTWYAPAVKG | SEQ ID NO: 4 | SAFTWSYGAADIAA | SEQ ID NO: 21 |
| | VL | SGRSGSYG | SEQ ID NO: 3 | DNTNRPS | SEQ ID NO: 5 | GGYWGSTDV | SEQ ID NO: 20 |
| 202 | VH | SFNMF | SEQ ID NO: 1 | EISGGGGSTWYAPAVKG | SEQ ID NO: 4 | SADTWSYGAADISA | SEQ ID NO: 22 |
| | VL | SGRSGSYG | SEQ ID NO: 3 | DNTNRPS | SEQ ID NO: 5 | GGYWGSTDV | SEQ ID NO: 20 |
| 203 | VH | SFNMF | SEQ ID NO: 1 | EISGGGGSTWYAPAVKG | SEQ ID NO: 4 | SADTWSYGAADIAA | SEQ ID NO: 23 |
| | VL | SGRSGSYG | SEQ ID NO: 3 | DNTNRPS | SEQ ID NO: 5 | GGYWGSTDV | SEQ ID NO: 20 |
| 204 | VH | SFNMF | SEQ ID NO: 1 | EISGGGGSTWYAPAVKG | SEQ ID NO: 4 | SADTWSYGAADISA | SEQ ID NO: 24 |
| | VL | SGRSGSYG | SEQ ID NO: 3 | DNTNRPS | SEQ ID NO: 5 | GGYWGSTDV | SEQ ID NO: 20 |
| 205 | VH | SFNMF | SEQ ID NO: 1 | EISGGGGSTWYAPAVKG | SEQ ID NO: 4 | SAFTWSYGAADIAA | SEQ ID NO:21 |
| | VL | SGRSGSYG | SEQ ID NO: 2 | DNTNRPS | SEQ ID NO: 5 | GGYWGSTDV | SEQ ID NO: 20 |
| 206 | VH | SFNMF | SEQ ID NO: 1 | EISGGGGSTWYAPAVKG | SEQ ID NO: 4 | SADTWSYGAADIQA | SEQ ID NO: 25 |
| | VL | SGRSGSYG | SEQ ID NO: 3 | DNTNRPS | SEQ ID NO: 5 | GGYWGSTDV | SEQ ID NO: 20 |
| 207 | VH | SFNMF | SEQ ID NO: 1 | EISGGGGSTWYAPAVKG | SEQ ID NO: 4 | SAFTWSYGAADIDA | SEQ ID NO:26 |
| | VL | SGRSGSYG | SEQ ID NO: 2 | DNTNRPS | SEQ ID NO: 5 | GGYWGSTDV | SEQ ID NO: 20 |
| 208 | VH | SFNMF | SEQ ID NO: 1 | EISGGGGSTWYAPAVKG | SEQ ID NO: 4 | SAFTWSYGAADISA | SEQ ID NO:27 |
| | VL | SGRSGSYG | SEQ ID NO: 2 | DNTNRPS | SEQ ID NO: 5 | GGYWGSTDV | SEQ ID NO: 20 |
| 209 | VH | SFNMF | SEQ ID NO: 1 | EISGGGGSTWYAPAVKG | SEQ ID NO: 4 | SAFTWSYGAADIDA | SEQ ID NO:26 |
| | VL | SGRSGSYG | SEQ ID NO: 3 | DNTNRPS | SEQ ID NO: 5 | GGYWGSTDV | SEQ ID NO: 20 |
| 210 | VH | SFNMF | SEQ ID NO: 1 | EISGGGGSTWYAPAVKG | SEQ ID NO: 4 | SADTWSYGAASISA | SEQ ID NO: 28 |
| | VL | SGRSGSYG | SEQ ID NO: 3 | DNTNRPS | SEQ ID NO: 5 | GGYWGSTDV | SEQ ID NO: 20 |
| 211 | VII | SFNMF | SEQ ID NO: 1 | EISGGGGSTWYAPAVKG | SEQ ID NO: 4 | SAFTWSYGAADISA | SEQ ID NO: 27 |
| | VL | SGRSGSYG | SEQ ID NO: 3 | DNTNRPS | SEQ ID NO: 5 | GGYWGSTDV | SEQ ID NO: 20 |
| 212 | VH | SFNMF | SEQ ID NO: 1 | EISGGGGSTWYAPAVKG | SEQ ID NO: 4 | SAFTWSYGAATIDA | SEQ ID NO:9 |
| | VL | SGRSGSYG | SEQ ID NO: 3 | DNTNRPS | SEQ ID NO: 5 | GGYWGSTDV | SEQ ID NO: 20 |
| 213 | VH | SFNMF | SEQ ID NO: 1 | EISGGGGSTWYAPAVKG | SEQ ID NO: 4 | SRDTWSYGAADIDA | SEQ ID NO:29 |
| | VL | SGRSGSYG | SEQ ID NO: 2 | DNTNRPS | SEQ ID NO: 5 | GGYWGSTDV | SEQ ID NO:20 |
| 214 | VH | SFNMF | SEQ ID NO: 1 | EISGGGGSTWYAPAVKG | SEQ ID NO: 4 | SAFTWSYGAASIQA | SEQ ID NO:30 |
| | VL | SGRSGSYG | SEQ ID NO: 2 | DNTNRPS | SEQ ID NO: 5 | GGYWGSTDV | SEQ ID NO: 20 |
| 215 | VH | SFNMF | SEQ ID NO: 1 | EISGGGGSTWYAPAVKG | SEQ ID NO: 4 | SADTWSYGAASIAA | SEQ ID NO: 31 |
| | VL | SGRSGSYG | SEQ ID NO: 2 | DNTNRPS | SEQ ID NO: 5 | GGYWGSTDV | SEQ ID NO: 20 |
| 216 | VII | SFNMF | SEQ ID NO: 1 | EISGGGGSTWYAPAVKG | SEQ ID NO: 4 | SRFTWSYGAADISA | SEQ ID NO: 32 |
| | VL | SGRSGSYG | SEQ ID NO: 3 | DNTNRPS | SEQ ID NO: 5 | GGYWGSTDV | SEQ ID NO: 20 |
| 217 | VH | SFNMF | SEQ ID NO: 1 | EISGGGGSTWYAPAVKG | SEQ ID NO: 4 | SAFTWSYGAATISA | SEQ ID NO:33 |
| | VL | SGRSGSYG | SEQ ID NO: 2 | DNTNRPS | SEQ ID NO: 5 | GGYWGSTDV | SEQ ID NO: 20 |
| 218 | VH | SFNMF | SEQ ID NO: 1 | EISGGGGSTWYAPAVKG | SEQ ID NO: 4 | SRFTWSYGAASIAA | SEQ ID NO:34 |
| | VL | SGRSGSYG | SEQ ID NO: 3 | DNTNRPS | SEQ ID NO: 5 | GGYWGSTDV | SEQ ID NO:20 |
| 219 | VH | SFNMF | SEQ ID NO: 1 | EISGGGGSTWYAPAVKG | SEQ ID NO: 4 | SAYTWSYGAATTYA | SEQ ID NO:35 |
| | VL | SGRSGSYG | SEQ ID NO: 2 | DNTNRPS | SEQ ID NO: 5 | GGYWGSTDV | SEQ ID NO: 20 |

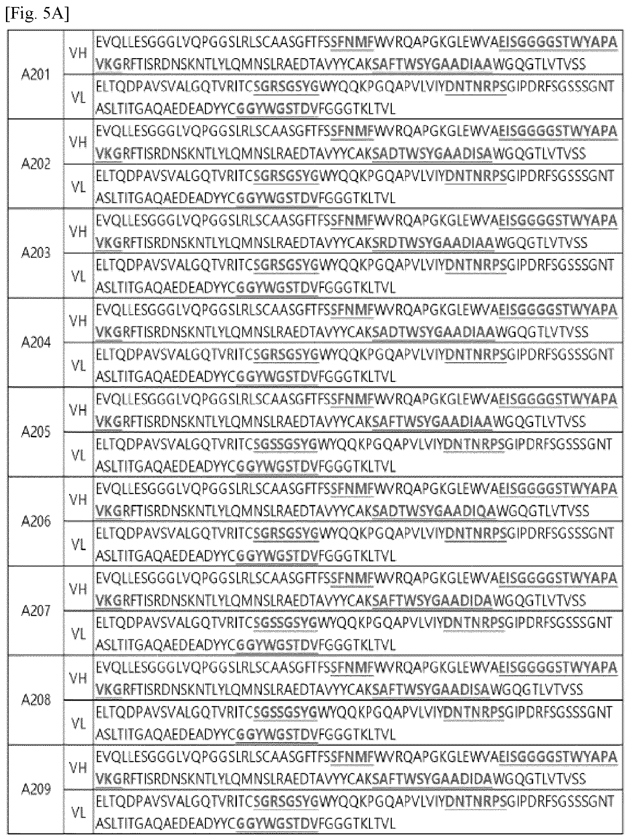

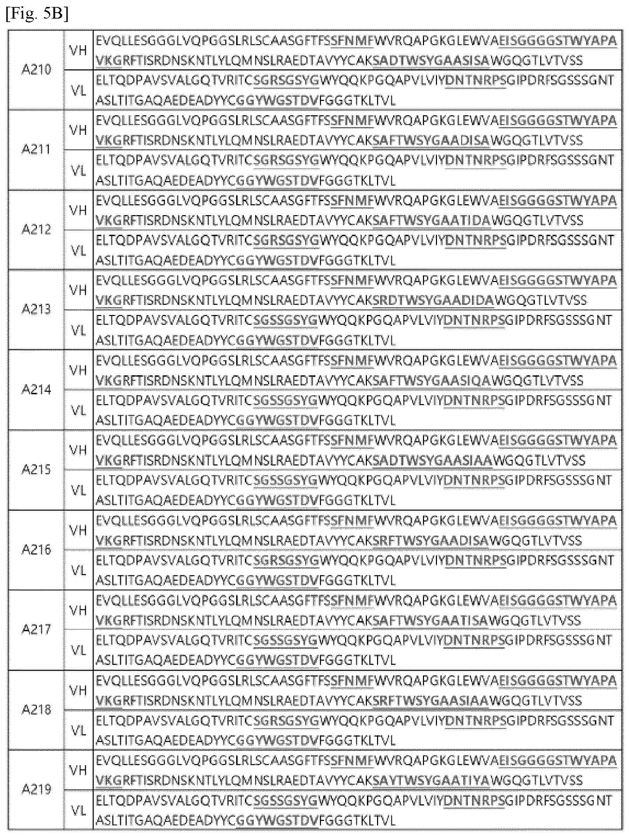

200 ng each of library DNAs for the CDRs in Table 7 were electroporated into E. coli BL21 and expressed as scFv fragments. The amino acid sequences of the heavy and light chain variable regions of each of the 19 scFvs are shown in FIG. 5 and Table 8 (The underlined and bolded sequences in FIG. 5 indicate CDR sequences in each region).

**[Table 8]**

| | | | |
|---|---|---|---|
| A201 | VH | SEQ ID NO: 52 | EVQLLESGGGLVQPGGSLRLSCAASGFTFSSFNMFWVRQAPGKGLE WVAEISGGGGSTWYAPAVKGRFTISRDNSKNTLYLQMNSLRAEDTA VYYCAKSAFTWSYGAADIAAWGQGTLVTVSS |
| | VL | SEQ ID NO: 53 | ELTQDPAVSVALGQTVRITCSGRSGSYGWYQQKPGQAPVLVIYDNTN RPSGIPDRFSGSSSGNTASLTITGAQAEDEADYYCGGYWGSTDVFGG GTKLTVL |
| A202 | VH | SEQ ID NO: 54 | EVQLLESGGGLVQPGGSLRLSCAASGFTFSSFNMFWVRQAPGKGLE WVAEISGGGGSTWYAPAVKGRFTISRDNSKNTLYLQMNSLRAEDTA VYYCAKSADTWSYGAADISAWGQGTLVTVSS |
| | VL | SEQ ID NO: 53 | ELTQDPAVSVALGQTVRITCSGRSGSYGWYQQKPGQAPVLVIYDNTN RPSGIPDRFSGSSSGNTASLTITGAQAEDEADYYCGGYWGSTDVFGG GTKLTVL |
| A203 | VH | SEQ ID NO: 55 | EVQLLESGGGLVQPGGSLRLSCAASGFTFSSFNMFWVRQAPGKGLE WVAEISGGGGSTWYAPAVKGRFTISRDNSKNTLYLQMNSLRAEDTA VYYCAKSRDTWSYGAADIAAWGQGTLVTVSS |
| | VL | SEQ ID NO: 53 | ELTQDPAVSVALGQTVRITCSGRSGSYGWYQQKPGQAPVLVIYDNTN RPSGIPDRFSGSSSGNTASLTITGAQAEDEADYYCGGYWGSTDVFGG GTKLTVL |
| A204 | VH | SEQ ID NO: 56 | EVQLLESGGGLVQPGGSLRLSCAASGFTFSSFNMFWVRQAPGKGLE WVAEISGGGGSTWYAPAVKGRFTISRDNSKNTLYLQMNSLRAEDTA VYYCAKSADTWSYGAADIAAWGQGTLVTVSS |
| | VL | SEQ ID NO: 53 | ELTQDPAVSVALGQTVRITCSGRSGSYGWYQQKPGQAPVLVIYDNTN RPSGIPDRFSGSSSGNTASLTITGAQAEDEADYYCGGYWGSTDVFGG GTKLTVL |
| A205 | VH | SEQ ID NO: 52 | EVQLLESGGGLVQPGGSLRLSCAASGFTFSSFNMFWVRQAPGKGLE WVAEISGGGGSTWYAPAKGRFTISRDNSKNTLYLQMNSLRAEDTAV YYCAKSAFTWSYGAADIAAWGQGTLVTVSS |
| | VL | SEQ ID NO: 51 | ELTQDPAVSVALGQTVRITCSGSSGSYGWYQQKPGQAPVLVIYDNTN RPSGIPDRFSGSSSGNTASLTITGAQAEDEADYYCGGYWGSTDVFGG GTKLTVL |
| A206 | VH | SEQ ID NO: 57 | EVQLLESGGGLVQPGGSLRLSCAASGFTFSSFNMFWVRQAPGKGLE WVAEISGGGGSTWYAPAVKGRFTISRDNSKNTLYLQMNSLRAEDTA VYYCAKSADTWSYGAADIQAWGQGTLVTVSS |
| | VL | SEQ ID NO: 53 | ELTQDPAVSVALGQTVRITCSGRSGSYGWYQQKPGQAPVLVIYDNTN RPSGIPDRFSGSSSGNTASLTITGAQAEDEADYYCGGYWGSTDVFGG GTKLTVL |
| A207 | VH | SEQ ID NO: 58 | EVQLLESGGGLVQPGGSLRLSCAASGFTFSSFNMFWVRQAPGKGLE WVAEISGGGGSTWYAPAVKGRFTISRDNSKNTLYLQMNSLRAEDTA VYYCAKSAFTWSYGAADIDAWGQGTLVTVSS |
| | VL | SEQ ID NO: 51 | ELTQDPAVSVALGQTVRITCSGSSGSYGWYQQKPGQAPVLVIYDNTN RPSGIPDRFSGSSSGNTASLTITGAQAEDEADYYCGGYWGSTDVFGG GTKLTVL |
| A208 | VH | SEQ ID NO: 59 | EVQLLESGGGLVQPGGSLRLSCAASGFTFSSFNMFWVRQAPGKGLE WVAEISGGGGSTWYAPAVKGRFTISRDNSKNTLYLQMNSLRAEDTA VYYCAKSAFTWSYGAADISAWGQGTLVTVSS |
| | VL | SEQ ID NO: 51 | ELTQDPAVSVALGQTVRITCSGSSGSYGWYQQKPGQAPVLVIYDNTN RPSGIPDRFSGSSSGNTASLTITGAQAEDEADYYCGGYWGSTDVFGG GTKLTVL |
| A209 | VH | SEQ ID NO: 58 | EVQLLESGGGLVQPGGSLRLSCAASGFTFSSFNMFWVRQAPGKGLE WVAEISGGGGSTWYAPAVKGRFTISRDNSKNTLYLQMNSLRAEDTA VYYCAKSAFTWSYGAADIDAWGQGTLVTVSS |
| | VL | SEQ ID NO: 53 | ELTQDPAVSVALGQTVRITCSGRSGSYGWYQQKPGQAPVLVIYDNTN RPSGIPDRFSGSSSGNT ASLTITGAQAEDEADYYCGGYWGSTDVFGGGTKLTVL |
| A210 | VH | SEQ ID NO: 60 | EVQLLESGGGLVQPGGSLRLSCAASGFTFSSFNMFWVRQAPGKGLE WVAEISGGGGSTWYAPAVKGRFTISRDNSKNTLYLQMNSLRAEDTA VYYCAKSADTWSYGAASISAWGQGTLVTVSS |
| | VL | SEQ ID NO: 53 | ELTQDPAVSVALGQTVRITCSGRSGSYGWYQQKPGQAPVLVIYDNTN RPSGIPDRFSGSSSGNTASLTITGAQAEDEADYYCGGYWGSTDVFGG GTKLTVL |
| A211 | VH | SEQ ID NO: 59 | EVQLLESGGGLVQPGGSLRLSCAASGFTFSSFNMFVWRQAPGKGLEV WAEISGGGGSTWYAPAVKGRFTISRDNSKNTLYLQMNSLRAEDTAV YYCAKSAFTWSYGAADISAWGQGTLVTVSS |
| | VL | SEQ ID NO: 53 | ELTQDPAVSVALGQTVRITCSGRSGSYGWYQQKPGQAPVLVIYDNTN RPSGIPDRFSGSSSGNTASLTITGAQAEDEADYYCGGYWGSTDVFGG GTKLTVL |
| A212 | VH | SEQ ID NO: 39 | EVQLLESGGGLVQPGGSLRLSCAASGFTFSSFNMFWVRQAPGKGLE WVAEISGGGGSTWYAPAVKGRFTISRDNSKNTLYLQMNSLRAEDTA VYYCAKSAFTWSYGAATIDAWGQGTLVTVSS |
| | VL | SEQ ID NO: 53 | ELTQDPAVSVALGQTVRITCSGRSGSYGWYQQKPGQAPVLVIYDNTN RPSGIPDRFSGSSSGNTASLTITGAQAEDEADYYCGGYWGSTDVFGG GTKLTVL |
| A213 | VH | SEQ ID NO: 61 | EVQLLESGGGLVQPGGSLRLSCAASGFTFSSFNMFWVRQAPGKGLE WVAEISGGGGSTWYAPAVKGRFTISRDNSKNTLYLQMNSLRAEDTA VYYCAKSRDTWSYGAADIDAWGQGTLVTVSS |
| | VL | SEQ ID NO: 51 | ELTQDPAVSVALGQTVRITCSGSSGSYGWYQQKPGQAPVLVIYDNTN RPSGIPDRFSGSSSGNT ASLTITGAQAEDEADYYCGGYWGSTDVFGGGTKLTVL |
| A214 | VH | SEQ ID NO: 62 | EVQLLESGGGLVQPGGSLRLSCAASGFTFSSFNMFWVRQAPGKGLE WVAEISGGGGSTWYAPAVKGRFTISRDNSKNTLYLQMNSLRAEDTA VYYCAKSAFTWSYGAASIQAWGQGTLVTVSS |
| | VL | SEQ ID NO: 51 | ELTQDPAVSVALGQTVRITCSGSSGSYGWYQQKPGQAPVLVIYDNTN RPSGIPDRFSGSSSGNT ASLTITGAQAEDEADYYCGGYWGSTDVFGGGTKLTVL |
| A215 | VH | SEQ ID NO: 63 | EVQLLESGGGLVQPGGSLRLSCAASGFTFSSFNMFWVRQAPGKGLE WVAEISGGGGSTWYAPAVKGRFTISRDNSKNTLYLQMNSLRAEDTA VYYCAKSADTWSYGAASIAAWGQGTLVTVSS |
| | VL | SEQ ID NO: 51 | ELTQDPAVSVALGQTVRITCSGSSGSYGWYQQKPGQAPVLVIYDNTN RPSGIPDRFSGSSSGNTASLTITGAQAEDEADYYCGGYWGSTDVFGG GTKLTVL |
| A216 | VH | SEQ ID NO: 64 | EVQLLESGGGLVQPGGSLRLSCAASGFTFSSFNMFWVRQAPGKGLE WVAEISGGGGSTWYAPAVKGRFTISRDNSKNTLYLQMNSLRAEDTA VYYCAKSRFTWSYGAADISAWGQGTLVTVSS |
| | VL | SEQ ID NO: 53 | ELTQDPAVSVALGQTVRITCSGRSGSYGWYQQKPGQAPVLVIYDNTN RPSGIPDRFSGSSSGNTASLTITGAQAEDEADYYCGGYWGSTDVFGG GTKLTVL |
| A217 | VH | SEQ ID NO: 65 | EVQLLESGGGLVQPGGSLRLSCAASGFTFSSFNMFWVRQAPGKGLE WVAEISGGGGSTWYAPAVKGRFTISRDNSKNTLYLQMNSLRAEDTA VYYCAKSAFTWSYGAATISAWGQGTLVTVSS |
| | VL | SEQ ID NO: 51 | ELTQDPAVSVALGQTVRITCSGSSGSYGWYQQKPGQAPVLVIYDNTN RPSGIPDRFSGSSSGNTASLTITGAQAEDEADYYCGGYWGSTDVFGG GTKLTVL |
| A218 | VH | SEQ ID NO: 66 | EVQLLESGGGLVQPGGSLRLSCAASGFTFSSFNMFWVRQAPGKGLE WVAEISGGGGSTWYAPAVKGRFTISRDNSKNTLYLQMNSLRAEDTA VYYCAKSRFTWSYGAASIAAWGQGTLVTVSS |
| | VL | SEQ ID NO: 53 | ELTQDPAVSVALGQTVRITCSGRSGSYGWYQQKPGQAPVLVIYDNTN RPSGIPDRFSGSSSGNTASLTITGAQAEDEADYYCGGYWGSTDVFGG GTKLTVL |
| A219 | VH | SEQ ID NO: 67 | EVQLLESGGGLVQPGGSLRLSCAASGFTFSSFNMFWVRQAPGKGLE WVAEISGGGGSTWYAPAVKGRFTISRDNSKNTLYLQMNSLRAEDTA VYYCAKSAYTWSYGAATIYAWGQGTLVTVSS |
| | VL | SEQ ID NO: 51 | ELTQDPAVSVALGQTVRITCSGSSGSYGWYQQKPGQAPVLVIYDNTN RPSGIPDRFSGSSSGNTASLTITGAQAEDEADYYCGGYWGSTDVFGG GTKLTVL |

### 4.2 Confirmation of CNTN4 binding affinity of scFv (2)

### Confirmation of binding affinity of scFv to antigen protein through FACS

An FACS test was performed in the same manner as in Example 1.2, except that the scFvs prepared in Example 4.1 were used and human CNTN4-overexpressing 293F cells were used.

As shown in Table 9, the results showed that all of the scFvs prepared in Example 4.1 exhibited high binding affinity to the CNTN4 protein at the nM level.

**[Table 9]**

| Sampple | FACS | |
|---|---|---|
| | human CNTN4 cell | |
| | Top MFI | EC50 (nM) |
| A201 | 1377.0 | 3.2 |
| A202 | 1752.0 | 3.0 |
| A203 | 1244.0 | 3.3 |
| A204 | 1696.0 | 1.7 |
| A205 | 1298.0 | 2.0 |
| A206 | 1369.0 | 4.8 |
| A207 | 1619.0 | 3.2 |
| A208 | 1315.0 | 3.7 |
| A209 | 1604.0 | 2.3 |
| A210 | 1381.0 | 4.4 |
| A211 | 1371.0 | 3.1 |
| A212 | 1630.0 | 2.7 |
| A213 | 1704.0 | 3.2 |
| A214 | 1456.0 | 3.5 |
| A215 | 1468.0 | 3.6 |
| A216 | 1626.0 | 2.9 |
| A217 | 1736.0 | 2.8 |
| A218 | 1294.0 | 3.7 |
| A219 | 1269.0 | 3.9 |
| NC | N/A | N/A |

### Example 5: Preparation of anti-CNTN4 IgG antibody (2)

Six of the scFvs prepared in Example 4.1 (A202, A204, A206, A209, A211, and A217) were selected to prepare antibodies.

Specifically, the VH genes of the selected six scFvs were fused with the CH1, hinge, CH2 and CH3 segments of heavy chain human IgG4 (S228P), and the VL genes thereof were fused with a light chain human Ig domain. The heavy chain and light chain plasmids were co-transfected into Expi293F cells. The cells were cultured for 6 hours, and the supernatant was collected. The supernatant of the cell culture was applied to a column through a sample inlet to purify a protein, and particularly, a Protein A column equilibrated with a wash buffer (100 mM Tris, pH 7.0) was used. After washing the column with 15 CV of the wash buffer, gradient elution was performed using buffer B (0.1 M glycine, pH 3.5). The protein concentration of the elution was determined by A280/extinction coefficient using Nanodrop 2000. By analyzing the purified protein through SDS-PAGE and HPLC-SEC, It was confirmed that an anti-CNTN4 antibody was prepared (Table 10).

The prepared antibody was named scFv-IgG antibody (e.g., the antibody prepared using A202 scFv was named A202-IgG antibody).

**[Table 10]**

| Antibody | Concentration (mg/mL) | HPLC-SEC Purity (%) |
|---|---|---|
| A202-IgG Ab | 8.53 | 94.5 |
| A204-IgG Ab | 7.42 | 97.8 |
| A206-IgG Ab | 6.27 | 95.2 |
| A209-TgG Ab | 9.60 | 96.7 |
| A211-IgG Ab | 8.01 | 95.0 |
| A217-IgG Ab | 11.47 | 98.8 |

### Example 6: Confirmation of CNTN4 binding affinity of anti-CNTN4 IgG antibody (2)

### 6.1 ELISA test

An ELISA test was performed on the six IgG antibodies (A202, A204, A206, A209, A211, and A217) prepared in Example 5. A 96-well plate was coated with anti-His antibody in a coating buffer at 4 °C overnight. Next day, the plate was blocked with 3% BSA in PBS at 25 °C for 1 hour. Human CNTN4 (His-tagged) was added to the plate and incubated at 25 °C for 1 hour. After washing, antibodies were added to the wells and incubated at 25 °C for 2 hours. Subsequently, the plate was incubated at 25 °C for 1 hour, together with an anti-human IgG (HRP) conjugate. HRP activity was detected with a tetramethylbenzidine (TMB) substrate, and the reaction was quenched with 2M HCl. The plate was read at 450 nm.

As shown in Table 11, the results showed that all of the six antibodies exhibited very high binding affinity, with EC50 values at the pM level.

**[Table 11]**

| Antibody | EC50 (nM) |
|---|---|
| A202-IgG Ab | 0.035 |
| A204-IgG Ab | 0.015 |
| A206-IgG Ab | 0.027 |
| A209-IgG Ab | 0.026 |
| A211-IgG Ab | 0.033 |
| A217-IgG Ab | 0.033 |

### 6.2 Surface Plasmon Resonance (SPR) test

An SPR test was performed on the six IgG antibodies (A202, A204, A206, A209, A211, and A217) prepared in Example 5.

Specifically, immediately before injection into a chip, an activating agent was prepared by mixing 400 mM EDC and 100 mM NHS (GE). A CM5 sensor chip was activated with the mixture at a flow rate of 10 µL/min for 420 seconds. 30 µg/mL of protein G in 10 mM NaAc (pH 4.0) was injected into the chip at a flow rate of 10 µL/min for 420 seconds. Afterward, the chip was inactivated by IM ethanolamine-HCl (GE) at a flow rate of 10 µL/min for 420 seconds.

IgG antibodies were captured on the chip using THETM His-tagged antibodies in running buffer lxHBS-EP+ at a flow rate of 10 µL/min. In the binding and dissociation stages, a series of concentrations of an analyte (human CNTN4 his-tagged) and a running buffer were sequentially injected into the chip at a flow rate of 30 µL/min.

The chip was regenerated with 10 mM glycine at pH 1.5.

A surface channel Fc1 without a captured ligand was used as a control surface for reference subtraction. The final data of each interaction was excluded from the data of reference Fc1 and buffer channels. The experimental data was fitted by 1:1 binding in the 8K (ver. 2.0.15.12933) evaluation software.

The measured equilibrium dissociation constant (K_{d}) is shown in Table 12. From the results, it was confirmed that the antibody of the present invention binds to the human CNTN4 protein with high affinity.

**[Table 12]**

| Antibody | K₀ (M) |
|---|---|
| A202-IgG Ab | 7.13 x 10¹⁰ |
| A204-IgG Ab | 6.63 x 10⁻¹⁰ |
| A206-IgG Ab | 7.02 x 10⁻¹⁰ |
| A209-IgG Ab | 6.62 x 10⁻¹⁰ |
| A211-IgG Ab | 7.09 x 10⁻¹⁰ |
| A217-IgG Ab | 1.18 x 10⁻⁹ |

### 6.3. FACS analysis

FACS analysis was performed on the IgG antibody A211 prepared in Example 5 along with the IgG antibody A114 prepared in Example 2. In addition, a human IgG4 antibody was used as a negative control (NC).

A HEK293 cell line that overexpresses human CNTN4 on the cell surface was manufactured. After seeding the overexpressing HEK293 cells at 1 x 10⁵ cells/well to each well of a 96-well plate, A114 and A211 antibodies pre-diluted to different concentrations were prepared and added to each well containing the cells. The highest concentration of the antibodies was set to 100 mg/mL, and serially diluted 3-fold to 14 points. After adding the antibodies, the cells were incubated at 4 °C for 1 hour. After washing the cells twice with a FACS buffer, an APC-conjugated anti-human IgG antibody was added to the cells, and the resulting cells were further incubated at 4 °C for 30 minutes. The cells were washed twice, and 7-AAD was added to each sample at a concentration of 0.5 mg/mL. Each sample was transferred to a FACS tube, and then the binding degrees of the A114 and A211 antibodies were analyzed using an FACS Canto II flow cytometer.

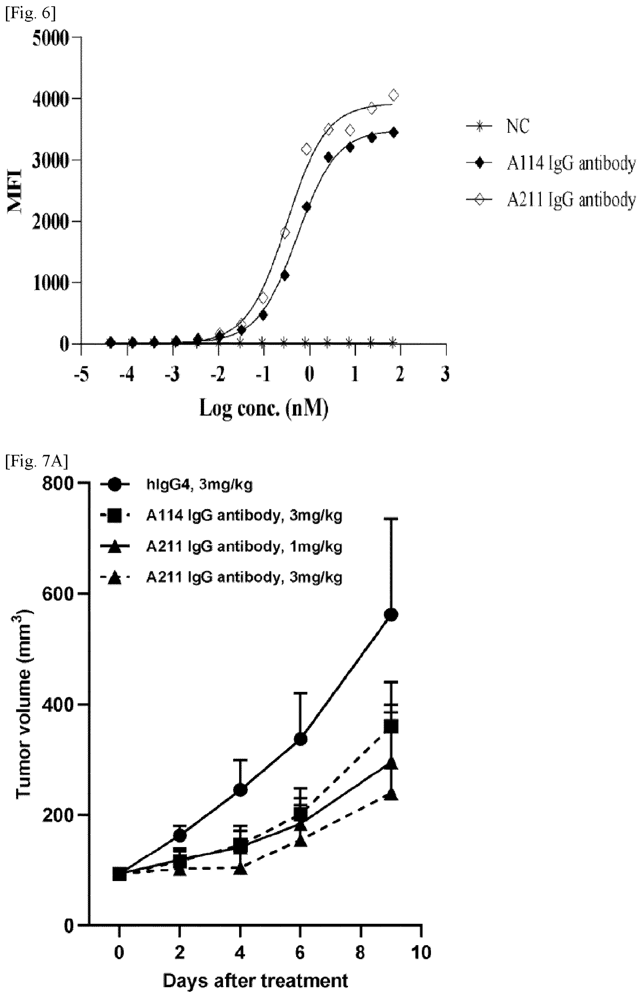

The results are shown in FIG. 6 and Table 13. It was observed that the mean fluorescence intensities (MFI) of both A114 and A211 were increased in a concentration-dependent manner (FIGS. 6), and the EC50 values thereof were observed to be 0.539 nM and 0.316 nM, respectively (Table 13).

**[Table 13]**

| Antibody | Top MFI | KC50 (nM) |
|---|---|---|
| Al14-IgG Ab | 3493 | 0.539 |
| A211-IgG Ab | 3924 | 0.316 |
| NC (hIgG4) | 13.02 | NA |

From the above results, it was confirmed that both antibodies A114 and A211 have high binding affinity to CNTN4 expressed on the cell surface.

### Example 7. Confirmation of tumor growth inhibition efficacy of antibody

In this example, it was intended to confirm the tumor growth inhibition efficacy of the antibody of the present invention in vivo.

This experiment was conducted with the IgG antibodies (A114 and A211) prepared in Examples 2 and 5, and CT26 syngeneic mouse tumor models were used. The CT26 tumor models were established by subcutaneously inoculating 1 X 10⁶ CT26 cancer cells into 6-week-old female BALB/c mice.

When the tumor volume reached 75 to 150 mm³, mice were randomly selected and divided into four groups of 10 mice each, and used to conduct the experiment. 1) A control (hIgG4, 3 mg/kg), 2) A114 IgG antibody (3 mg/kg), 3) A211 IgG antibody (1 mg/kg), and 4) A211 IgG antibody (3 mg/kg) were intraperitoneally administered to each group every 3 days (q3d), and the administration was performed four times (D0, D3, D6, and D9) over 2 weeks. The growing tumor size was measured three times a week, and the body weight of the mouse was measured twice a week. The tumor inhibition efficacy of the antibodies was evaluated relative to the control, by setting the tumor growth rate of the control (human IgG4) at the final drug administration (D9) to 100% (relative tumor growth, 100%).

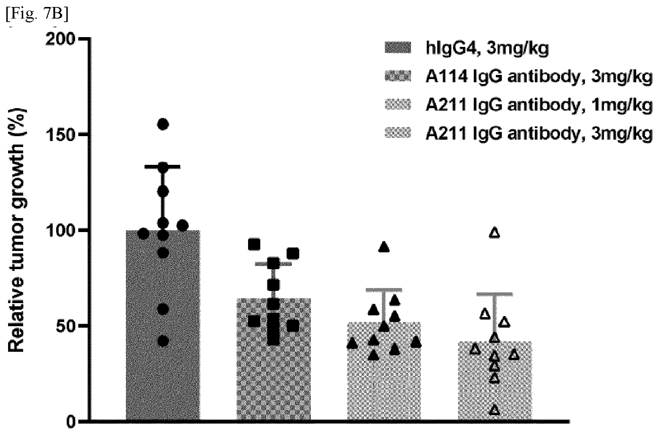

The results are shown in FIG. 7 and Table 14.

**[Table 14]**

| **Group (dose level mg/kg)** | **Tumor volume (Mean, mm³)** | | | | | **Tumor growth inhibition on Day 9 (%)** |
|---|---|---|---|---|---|---|
| | Day 0 | Day 2 | Day 4 | Day 6 | Day 9 | |
| hIgG4 (3 mg/kg) | 93.70 | 163.02 | 245.30 | 337.24 | 562.02 | |
| A114-IgG Ab (3 mg/kg) | 93.72 | 116.24 | 146.01 | 201.26 | 360.25 | 35.58 |
| A211-IgG Ab (1 mg/kg) | 93.70 | 118.64 | 141.89 | 184.05 | 295.94 | 48.10 |
| A211-IgG Ab (3 mg/kg) | 93.62 | 102.56 | 105.16 | 155.11 | 239.63 | 58.00 |

In the cases of A114 and A211 administration, compared to the control (hIgG4), tumor growth was statistically significantly inhibited, and A211 administration exhibited drug dose-dependent efficacy (FIG. 7A). When the tumor growth rate of the control at the final administration (D9) was set to 100%, the A114 (3 mg/kg) group exhibited a tumor growth rate of approximately 64.4%, the A211 (1 mg/kg) group exhibited a tumor growth rate of approximately 51.9%, and the A211 (3 mg/kg) group exhibited a tumor growth rate of approximately 42% (FIG. 7B). That is, compared to the control, the A114 (3 mg/kg) group exhibited a tumor growth inhibition rate of approximately 35.6%, the A211 (1 mg/kg) group and A211 (3 mg/kg) group exhibited a tumor growth inhibition rate of approximately 48.1% and 58.0 %, respectively (Table. 14). No adverse reactions including body weight changes were observed.

From the above results, it was confirmed that the antibodies of the present invention exhibited tumor inhibition efficacy without adverse reactions in vivo.

## Claims

1. An anti-CNTN4 antibody or an antigen-binding fragment thereof, comprising:
heavy chain CDR1 having the amino acid sequence of SEQ ID NO: 1;
heavy chain CDR2 having the amino acid sequence of SEQ ID NO: 4;
heavy chain CDR3 having any one amino acid sequence selected from the group consisting of SEQ ID NOs: 6, 8 to 18, and 21 to 35;
light chain CDR1 having the amino acid sequence of SEQ ID NO: 2 or 3;
light chain CDR2 having the amino acid sequence of SEQ ID NO: 5; and
light chain CDR3 having any one amino acid sequence selected from the group consisting of SEQ ID NOs: 7, 19, and 20.

2. The anti-CNTN4 antibody or an antigen-binding fragment thereof of claim 1, which comprises
101) heavy chain CDR1, heavy chain CDR2, heavy chain CDR3, light chain CDR1, light chain CDR2, and light chain CDR3, which have the amino acid sequences of SEQ ID NOs: 1, 4, 6, 2, 5, and 7, respectively;
102) heavy chain CDR1, heavy chain CDR2, heavy chain CDR3, light chain CDR1, light chain CDR2, and light chain CDR3, which have the amino acid sequences of SEQ ID NOs: 1, 4, 8, 2, 5, and 7, respectively;
103) heavy chain CDR1, heavy chain CDR2, heavy chain CDR3, light chain CDR1, light chain CDR2, and light chain CDR3, which have the amino acid sequences of SEQ ID NOs: 1, 4, 9, 2, 5, and 7, respectively;
104) heavy chain CDR1, heavy chain CDR2, heavy chain CDR3, light chain CDR1, light chain CDR2, and light chain CDR3, which have the amino acid sequences of SEQ ID NOs: 1, 4, 10, 2, 5, and 7, respectively;
105) heavy chain CDR1, heavy chain CDR2, heavy chain CDR3, light chain CDR1, light chain CDR2, and light chain CDR3, which have the amino acid sequences of SEQ ID NOs: 1,4, 11, 2, 5, and 7, respectively;
106) heavy chain CDR1, heavy chain CDR2, heavy chain CDR3, light chain CDR1, light chain CDR2, and light chain CDR3, which have the amino acid sequences of SEQ ID NOs: 1, 4, 12, 2, 5, and 7, respectively;
107) heavy chain CDR1, heavy chain CDR2, heavy chain CDR3, light chain CDR1, light chain CDR2, and light chain CDR3, which have the amino acid sequences of SEQ ID NOs: 1, 4, 13, 2, 5, and 7, respectively;
108) heavy chain CDR1, heavy chain CDR2, heavy chain CDR3, light chain CDR1, light chain CDR2, and light chain CDR3, which have the amino acid sequences of SEQ ID NOs: 1, 4, 14, 2, 5, and 7, respectively;
109) heavy chain CDR1, heavy chain CDR2, heavy chain CDR3, light chain CDR1, light chain CDR2, and light chain CDR3, which have the amino acid sequences of SEQ ID NOs: 1, 4, 15, 2, 5, and 7, respectively;
110) heavy chain CDR1, heavy chain CDR2, heavy chain CDR3, light chain CDR1, light chain CDR2, and light chain CDR3, which have the amino acid sequences of SEQ ID NOs: 1,4, 16, 2, 5, and 7, respectively;
111) heavy chain CDR1, heavy chain CDR2, heavy chain CDR3, light chain CDR1, light chain CDR2, and light chain CDR3, which have the amino acid sequences of SEQ ID NOs: 1, 4, 17, 2, 5, and 7, respectively;
112) heavy chain CDR1, heavy chain CDR2, heavy chain CDR3, light chain CDR1, light chain CDR2, and light chain CDR3, which have the amino acid sequences of SEQ ID NOs: 1, 4, 18, 3, 5, and 7, respectively;
113) heavy chain CDR1, heavy chain CDR2, heavy chain CDR3, light chain CDR1, light chain CDR2, and light chain CDR3, which have the amino acid sequences of SEQ ID NOs: 1, 4, 18, 2, 5, and 19, respectively;
114) heavy chain CDR1, heavy chain CDR2, heavy chain CDR3, light chain CDR1, light chain CDR2, and light chain CDR3, which have the amino acid sequences of SEQ ID NOs: 1, 4, 18, 2, 5, and 20, respectively;
201) heavy chain CDR1, heavy chain CDR2, heavy chain CDR3, light chain CDR1, light chain CDR2, and light chain CDR3, which have the amino acid sequences of SEQ ID NOs: 1, 4, 21, 3, 5, and 20, respectively;
202) heavy chain CDR1, heavy chain CDR2, heavy chain CDR3, light chain CDR1, light chain CDR2, and light chain CDR3, which have the amino acid sequences of SEQ ID NOs: 1, 4, 22, 3, 5, and 20, respectively;
203) heavy chain CDR1, heavy chain CDR2, heavy chain CDR3, light chain CDR1, light chain CDR2, and light chain CDR3, which have the amino acid sequences of SEQ ID NOs: 1, 4, 23, 3, 5, and 20, respectively;
204) heavy chain CDR1, heavy chain CDR2, heavy chain CDR3, light chain CDR1, light chain CDR2, and light chain CDR3, which have the amino acid sequences of SEQ ID NOs: 1, 4, 24, 3, 5, and 20, respectively;
205) heavy chain CDR1, heavy chain CDR2, heavy chain CDR3, light chain CDR1, light chain CDR2, and light chain CDR3, which have the amino acid sequences of SEQ ID NOs: 1, 4, 21, 2, 5, and 20, respectively;
206) heavy chain CDR1, heavy chain CDR2, heavy chain CDR3, light chain CDR1, light chain CDR2, and light chain CDR3, which have the amino acid sequences of SEQ ID NOs: 1, 4, 25, 3, 5, and 20, respectively;
207) heavy chain CDR1, heavy chain CDR2, heavy chain CDR3, light chain CDR1, light chain CDR2, and light chain CDR3, which have the amino acid sequences of SEQ ID NOs: 1, 4, 26, 2, 5, and 20, respectively;
208) heavy chain CDR1, heavy chain CDR2, heavy chain CDR3, light chain CDR1, light chain CDR2, and light chain CDR3, which have the amino acid sequences of SEQ ID NOs: 1, 4, 27, 2, 5, and 20, respectively;
209) heavy chain CDR1, heavy chain CDR2, heavy chain CDR3, light chain CDR1, light chain CDR2, and light chain CDR3, which have the amino acid sequences of SEQ ID NOs: 1, 4, 26, 3, 5, and 20, respectively;
210) heavy chain CDR1, heavy chain CDR2, heavy chain CDR3, light chain CDR1, light chain CDR2, and light chain CDR3, which have the amino acid sequences of SEQ ID NOs: 1, 4, 28, 3, 5, and 20, respectively;
211) heavy chain CDR1, heavy chain CDR2, heavy chain CDR3, light chain CDR1, light chain CDR2, and light chain CDR3, which have the amino acid sequences of SEQ ID NOs: 1, 4, 27, 3, 5, and 20, respectively;
212) heavy chain CDR1, heavy chain CDR2, heavy chain CDR3, light chain CDR1, light chain CDR2, and light chain CDR3, which have the amino acid sequences of SEQ ID NOs: 1, 4, 9, 3, 5, and 20, respectively;
213) heavy chain CDR1, heavy chain CDR2, heavy chain CDR3, light chain CDR1, light chain CDR2, and light chain CDR3, which have the amino acid sequences of SEQ ID NOs: 1, 4, 29, 2, 5, and 20, respectively;
214) heavy chain CDR1, heavy chain CDR2, heavy chain CDR3, light chain CDR1, light chain CDR2, and light chain CDR3, which have the amino acid sequences of SEQ ID NOs: 1, 4, 30, 2, 5, and 20, respectively;
215) heavy chain CDR1, heavy chain CDR2, heavy chain CDR3, light chain CDR1, light chain CDR2, and light chain CDR3, which have the amino acid sequences of SEQ ID NOs: 1, 4, 31, 2, 5, and 20, respectively;
216) heavy chain CDR1, heavy chain CDR2, heavy chain CDR3, light chain CDR1, light chain CDR2, and light chain CDR3, which have the amino acid sequences of SEQ ID NOs: 1, 4, 32, 3, 5, and 20, respectively;
217) heavy chain CDR1, heavy chain CDR2, heavy chain CDR3, light chain CDR1, light chain CDR2, and light chain CDR3, which have the amino acid sequences of SEQ ID NOs: 1, 4, 33, 2, 5, and 20, respectively;
218) heavy chain CDR1, heavy chain CDR2, heavy chain CDR3, light chain CDR1, light chain CDR2, and light chain CDR3, which have the amino acid sequences of SEQ ID NOs: 1, 4, 34, 3, 5, and 20, respectively; or
219) heavy chain CDR1, heavy chain CDR2, heavy chain CDR3, light chain CDR1, light chain CDR2, and light chain CDR3, which have the amino acid sequences of SEQ ID NOs: 1, 4, 35, 2, 5, and 20, respectively.

3. The anti-CNTN4 antibody or an antigen-binding fragment thereof of claim 1, which comprises
a heavy chain variable region having any one amino acid sequence selected from the group consisting of SEQ ID NOs: 36, 38 to 48, 52, and 54 to 67; and a light chain variable region having any one amino acid sequence selected from the group consisting of SEQ ID NOs: 37, 49 to 51, and 53.

4. The anti-CNTN4 antibody or an antigen-binding fragment thereof of claim 1, which comprises
A101) a heavy chain variable region and a light chain variable region, which have the amino acid sequences of SEQ ID NOs: 36 and 37, respectively;
A102) a heavy chain variable region and a light chain variable region, which have the amino acid sequences of SEQ ID NOs: 38 and 37, respectively;
A103) a heavy chain variable region and a light chain variable region, which have the amino acid sequences of SEQ ID NOs: 39 and 37, respectively;
A104) a heavy chain variable region and a light chain variable region, which have the amino acid sequences of SEQ ID NOs: 40 and 37, respectively;
A105) a heavy chain variable region and a light chain variable region, which have the amino acid sequences of SEQ ID NOs: 41 and 37, respectively;
A106) a heavy chain variable region and a light chain variable region, which have the amino acid sequences of SEQ ID NOs: 42 and 37, respectively;
A107) a heavy chain variable region and a light chain variable region, which have the amino acid sequences of SEQ ID NOs: 43 and 37, respectively;
A108) a heavy chain variable region and a light chain variable region, which have the amino acid sequences of SEQ ID NOs: 44 and 37, respectively;
A109) a heavy chain variable region and a light chain variable region, which have the amino acid sequences of SEQ ID NOs: 45 and 37, respectively;
A110) a heavy chain variable region and a light chain variable region, which have the amino acid sequences of SEQ ID NOs: 46 and 37, respectively;
A111) a heavy chain variable region and a light chain variable region, which have the amino acid sequences of SEQ ID NOs: 47 and 37, respectively;
A112) a heavy chain variable region and a light chain variable region, which have the amino acid sequences of SEQ ID NOs: 48 and 49, respectively;
A113) a heavy chain variable region and a light chain variable region, which have the amino acid sequences of SEQ ID NOs: 48 and 50, respectively;
A114) a heavy chain variable region and a light chain variable region, which have the amino acid sequences of SEQ ID NOs: 48 and 51, respectively;
A201) a heavy chain variable region and a light chain variable region, which have the amino acid sequences of SEQ ID NOs: 52 and 53, respectively;
A202) a heavy chain variable region and a light chain variable region, which have the amino acid sequences of SEQ ID NOs: 54 and 53, respectively;
A203) a heavy chain variable region and a light chain variable region, which have the amino acid sequences of SEQ ID NOs: 55 and 53, respectively;
A204) a heavy chain variable region and a light chain variable region, which have the amino acid sequences of SEQ ID NOs: 56 and 53, respectively;
A205) a heavy chain variable region and a light chain variable region, which have the amino acid sequences of SEQ ID NOs: 52 and 51, respectively;
A206) a heavy chain variable region and a light chain variable region, which have the amino acid sequences of SEQ ID NOs: 57 and 53, respectively;
A207) a heavy chain variable region and a light chain variable region, which have the amino acid sequences of SEQ ID NOs: 58 and 51, respectively;
A208) a heavy chain variable region and a light chain variable region, which have the amino acid sequences of SEQ ID NOs: 59 and 51, respectively;
A209) a heavy chain variable region and a light chain variable region, which have the amino acid sequences of SEQ ID NOs: 58 and 53, respectively;
A210) a heavy chain variable region and a light chain variable region, which have the amino acid sequences of SEQ ID NOs: 60 and 53, respectively;
A211) a heavy chain variable region and a light chain variable region, which have the amino acid sequences of SEQ ID NOs: 59 and 53, respectively;
A212) a heavy chain variable region and a light chain variable region, which have the amino acid sequences of SEQ ID NOs: 39 and 53, respectively;
A213) a heavy chain variable region and a light chain variable region, which have the amino acid sequences of SEQ ID NOs: 61 and 51, respectively;
A214) a heavy chain variable region and a light chain variable region, which have the amino acid sequences of SEQ ID NOs: 62 and 51, respectively;
A215) a heavy chain variable region and a light chain variable region, which have the amino acid sequences of SEQ ID NOs: 63 and 51, respectively;
A216) a heavy chain variable region and a light chain variable region, which have the amino acid sequences of SEQ ID NOs: 64 and 53, respectively;
A217) a heavy chain variable region and a light chain variable region, which have the amino acid sequences of SEQ ID NOs: 65 and 51, respectively;
A218) a heavy chain variable region and a light chain variable region, which have the amino acid sequences of SEQ ID NOs: 66 and 53, respectively; or
A219) a heavy chain variable region and a light chain variable region, which have the amino acid sequences of SEQ ID NOs: 67 and 51, respectively.

5. The anti-CNTN4 antibody or an antigen-binding fragment thereof of any one of claims 1 to 4, which specifically binds to the CNTN4 protein.

6. The anti-CNTN4 antibody or an antigen-binding fragment thereof of any one of claims 1 to 4, which binds to the CNTN4 protein with an equilibrium dissociation constant (KD) of 1 x 10-8 M or less.

7. The anti-CNTN4 antibody or an antigen-binding fragment thereof of any one of claims 1 to 4, which binds to the CNTN4 protein with an EC50 of 100 nM or less.

8. The anti-CNTN4 antibody or an antigen-binding fragment thereof of any one of claims 1 to 4, wherein the antigen-binding fragment is Fab, Fab', Fab'-SH, Fv, a single-chain variable fragment (scFv), an F(ab')2 fragment, VL, VH, a diabody, a triabody, a tetrabody, a minibody ((scFV-CH3)2), IgG ΔCH2, scFv-Fc, (scFv)2-Fc, a fynomer, dual-affinity re-targeting (DART), or TRIDENT.

9. The anti-CNTN4 antibody or an antigen-binding fragment thereof of any one of claims 1 to 4, wherein the antibody is a chimeric antibody, a humanized antibody, or a human antibody.

10. An antibody-drug conjugate comprising the anti-CNTN4 antibody or an antigen-binding fragment thereof of any one of claims 1 to 4.

11. A nucleic acid molecule that encodes the anti-CNTN4 antibody or an antigen-binding fragment thereof of any one of claims 1 to 4.

12. A recombinant expression vector comprising the nucleic acid molecule of claim 11.

13. A pharmaceutical composition for preventing or treating cancer, comprising the anti-CNTN4 antibody or an antigen-binding fragment thereof of any one of claims 1 to 4.

14. The pharmaceutical composition of claim 13, wherien the cancer is cancer that expresses CNTN4.

15. A pharmaceutical composition for preventing or treating cancer, comprising:
the anti-CNTN4 antibody or an antigen-binding fragment thereof of any one of claims 1 to 4; and an additional anti-cancer agent.

16. The pharmaceutical composition of claim 15, wherein the additional anti-cancer agent is an immune checkpoint inhibitor or a chemotherapeutic agent.

17. The pharmaceutical composition of claim 16, wherein the immune checkpoint inhibitor is one or more selected from the group consisting of an anti-CTLA-4 antibody, an anti-PD-1 antibody, and an anti-PD-L1 antibody.

18. The pharmaceutical composition of claim 15, wherein the anti-CNTN4 antibody or an antigen-binding fragment thereof and the additional anti-cancer agent are simultaneously administered as one preparation, or simultaneously or sequentially administered as separate preparations.

19. A pharmaceutical composition for preventing or treating cancer, comprising: the anti-CNTN4 antibody or an antigen-binding fragment thereof of any one of claims 1 to 4, which is used together with an additional anticancer therapy.

20. The pharmaceutical composition of claim 19, wherein the additional anticancer therapy is one or more selected from the group consisting of an immune checkpoint inhibitor, a chemotherapeutic agent, and radiation therapy.

21. A method of preventing or treating cancer in a subject, comprising: administering the anti-CNTN4 antibody or an antigen-binding fragment thereof of any one of claims 1 to 4 to a subject.

22. A use of the anti-CNTN4 antibody or an antigen-binding fragment thereof of any one of claims 1 to 4 for preparing a drug for preventing or treating cancer.
